# FASCICULE DE BREVET EUROPEEN

(11) **EP 1 720 872 B1**
(45) Date de publication et mention de la délivrance du brevet: **20.04.2011**
(21) Numéro de dépôt: 05732910.4
(22) Date de dépôt: 25.02.2005
(51) Int. Cl.: C07D 471/04, C07D 401/04, C07D 211/52, C07D 211/36, C07D 417/04, C07D 413/04, C07D 401/06, A61K 31/4523

(54) **DERIVES D'ARYL- ET D'HETEROARYL-PIPERIDINECARBOXYLATES, LEUR PREPARATION ET LEUR APPLICATION COMME INHIBITEURS DE L'ENZYME FAAH**
ARYL- UND HETEROARYLPIPERIDINCARBOXYLATDERIVATE, DEREN HERSTELLUNG UND DEREN VERWENDUNG IN FORM VON FAAH-ENZYMINHIBITOREN
ARYL AND HETEROARYL-PIPERIDINECARBOXYLATE DERIVATIVES, THE PREPARATION AND THE USE THEREOF IN THE FORM OF FAAH ENZYME INHIBITORS

(30) Priorité: 26.02.2004 FR 0401950
(43) Date de publication de la demande: 15.11.2006
(73) Titulaire: Sanofi-Aventis, 75013 Paris (FR)
(72) Inventeur: ABOUABDELLAH, Ahmed, F-94320 Thiais (FR); ALMARIO GARCIA, Antonio, F-92290 Chatenay Malabry (FR); HOORNAERT, Christian, F-92160 Antony (FR); LARDENOIS, Patrick, F-92340 Bourg-la-Reine (FR); MARGUET, Frank, F-91370 Verrières le Buisson (FR)
(74) Mandataire: Tanty, François
(86) Numéro de dépôt international: PCT/FR2005/000453
(87) Numéro de publication internationale: WO 2005/090347

(56) Documents cités:
- WO-A-99/26584
- WO-A-02/087569
- WO-A-2004/067498
- WO-A-2004/099176
- WO-A2-2004/020430
- TARZIA G ET AL: "Design, synthesis, and structure-activity relationships of alkylcarbamic acid aryl esters, a new class of Fatty Acid Amide Hydrolase inhibitors" JOURNAL OF MEDICINAL AND PHARMACEUTICAL CHEMISTRY, AMERICAN CHEMICAL SOCIETY. EASTON, US, vol. 46, no. 12, 2003, pages 2352-2360, XP002257137

## Description

L'invention a pour objet des dérivés d'aryl- et d'hétéroaryl-pipéridinecarboxylates, leur préparation et leur application en thérapeutique.

On connaît déjà des dérivés de phénylalkylcarbamates, de dioxane-2-alkylcarbamates et de type aryloxyalkylcarbamates, décrits respectivement dans les documents FR2850377 A, WO2004/020430 A2 et WO2005/077898, inhibiteurs de l'enzyme FAAH (Fatty Acid Amido Hydrolase).

Il existe toujours une nécessité de trouver et de développer des produits inhibiteurs de l'enzyme FAAH. Les composés de l'invention répondent à ce but.

Les composés de l'invention répondent à la formule générale (I) : 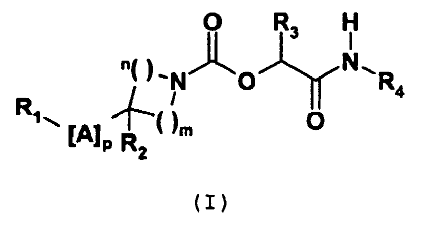 choisi parmi les composés du tableau suivant :

| n° | R₁ | **[A] ₚ** | **R₂** | **n** | **m** | **R₃** | **R₄** |
|---|---|---|---|---|---|---|---|
| 1. | phényl | liaison | H | 2 | 2 | H | H |
| 2. | phényl | liaison | H | 2 | 2 | H | CH₃ |
| 3. | 3-CF₃-phényl | liaison | H | 2 | 2 | H | H |
| 4. | 3-CF₃-phényl | liaison | H | 2 | 2 | H | CH₃ |
| 5. | 5-iso-butyl-pyridin-2-yl | liaison | H | 2 | 2 | H | CH₃ |
| 6. | 6-iso-butyl-pyridin-2-yl | liaison | H | 2 | 2 | H | CH₃ |
| 7. | 6-cyclopentyl-pyridin-2-yl | liaison | H | 2 | 2 | H | CH₃ |
| 8. | 5-(4-F-phényl)-pyridin-2-yl | liaison | H | 2 | 2 | H | CH₃ |
| 9. | 6-(4-F-phényl)-pyridin-2-yl | liaison | H | 2 | 2 | H | CH₃ |
| 10. | 6-(4-Cl-phényl)-pyridin-2-yl | liaison | H | 2 | 2 | H | CH₃ |
| 11. | 5-(4-CF₃-phényl)-pyridin-2-yl | liaison | H | 2 | 2 | H | CH₃ |
| 12. | 6-(4-CF₃-phényl)-pyridin-2-yl | liaison | H | 2 | 2 | H | CH₃ |
| 13. | 5-(3-CF₃-phényl)-1-méthyl-pyrazol-3-yl | liaison | H | 2 | 2 | H | CH₃ |
| 14. | 4-phényl-imidazol-1-yl | liaison | H | 2 | 2 | H | CH₃ |
| 15. | 5-phénys-1,3,9-oxadiazol--2-yl | liaison | H | 2 | 2 | H | H |
| 16. | 5-phényl-1,3,9-oxadiazol-2-yl | liaison | H | 2 | 2 | H | CH₃ |
| 17 . | 5-(4-F-phényl)-1,3,4-oxadiazol-2-yl | liaison | H | 2 | 2 | H | H |
| 18. | 5-(4-F-phényl)-1,3,4-oxadiazol-2-yl | liaison | H | 2 | 2 | H | CH₃ |
| 19. | 5- (3-CF₃-phényl)-1,3,4-oxadiazol-2-yl | liaison | H | 2 | 2 | H | H |
| 20. | 5-(3-CF₃-phényl)-1,3,4-oxadiazol-2-yl | liaison | H | 2 | 2 | H | CH₃ |
| 21. | 3-(3-CF₃-phényl)-1,2,4-oxadiazol-5-yl | liaison | H | 2 | 2 | H | H |
| 22. | 3- (3-CF₃-phényl)-1,2,4-oxadiazol-5-yl | liaison | H | 2 | 2 | H | CH₃ |
| 23. | benzoxazol-2-yl | liaison | H | 2 | 2 | H | CH₃ |
| 24. | benzothiazol-2-yl | liaison | H | 2 | 2 | H | H |
| 25. | benzothiazol-2-yl | liaison | H | 2 | 2 | H | CH₃ |
| 26. | benzimidazol-2-yl | liaison | H | 2 | 2 | H | CH₃ |
| 27. | benzimidazol-1-yl | liaison | H | 2 | 2 | H | H |
| 28. | 2-phényl-benzimidazol-1-yl | liaison | H | 2 | 2 | H | CH₃ |
| 29. | benzotriazol-1-yl | liaison | H | 2 | 2 | H | CH₃ |
| 30. | 5-CF₃-benzotriazol-1-yl | liaison | H | 2 | 2 | H | H |
| 31. | indol-1-yl | liaison | H | 2 | 2 | H | H |
| 32. | 4-Br-phényl | liaison | OH | 2 | 2 | H | CH₃ |
| 33. | 4-(4-F-phényl)phényl | liaison | OH | 2 | 2 | H | CH₃ |
| 34. | 4-(4-C1-phényl)phényl | liaison | OH | 2 | 2 | H | CH_{3.} |
| 35. | 4-(4-CH₃-phényl)phényl | liaison | OH | 2 | 2 | H | CH₃ |
| 36. | 4-(4-n-butyl-phényl) phényl | liaison | OH | 2 | 2 | H | CH₃ |
| 37. | 4-(4-CF₃-phényl) phényl | liaison | OH | 2 | 2 | H | CH₃ |
| 38. | 4-(4-CH₃O-phényl) phényl | liaison | OH | 2 | 2 | H | CH₃ |
| 39. | 4-(4-C₂H₅O-phényl) phényl | liaison | OH | 2 | 2 | H | CH₃ |
| 40. | 4-(3-C1,4-C1-phényl) phényl | liaison | OH | 2 | 2 | H | CH₃ |
| 41. | 4-(3-F,4-CH₃O-phényl) phényl | liaison | OH | 2 | 2 | H | CH₃ |
| 42. | 9-(3-C1,4-F-phényl) phényl | liaison | OH | 2 | 2 | H | CH₃ |
| 43. | naphthalèn-2-yl | CH₂ | H | 2 | 2 | H | CH₃ |
| 44. | 4-phényl-phényl | CH₂ | H | 2 | 2 | H | CH₃ |
| 45. | 6-cyclopentyl-pyridin-2-yl | CH₂ | H | 2 | 2 | H | CH₃ |
| 46. | 6-(4-F-phényl)-pyridin-2-yl | CH₂ | H | 2 | 2 | H | CH₃ |
| 47. | indol-1-yl | CH₂ | H | 2 | 2 | H | H |
| 48. | indolin-1-yl | CH₂ | H | 2 | 2 | H | H |
| 49. | 1,2,3,4-tétrahydroquinolin-1-yl | CH₂ | H | 2 | 2 | H | H |
| 50. | 1,2,3,4-tétrahydroisoquinolin -2-yl | CH₂ | H | 2 | 2 | H | H |
| 51. | pyrrolo [2,3-b] pyridin-1-yl | CH₂ | H | 2 | 2 | H | H |
| 52. | benzimidazol-1-yl | CH₂ | H | 2 | 2 | H | H |
| 53. | 4-phénylimidazol-1-yl | CH₂ | H | 2 | 2 | H | H |
| 54. | phényl | (CH₂) ₂ | H | 1 | 2 | H | CH₃ |
| 55. | 4-F-phényl | (CH₂) ₂ | H | 2 | 2 | H | CH₃ |
| 56. | 3-Cl-phényl | (CH₂) ₂ | H | 2 | 2 | H | CH₃ |
| 57. | 4-Cl-phényl | (CH₂) ₂ | H | 2 | 2 | H | CH₃ |
| 58. | 3-CF₃-phényl | (CH₂) ₂ | H | 2 | 2 | H | CH₃ |
| 59. | 4-CF₃-phényl | (CH₂)₂ | H | 2 | 2 | H | CH₃ |
| 60. | 3-CN-phényl | (CH₂)₂ | H | 2 | 2 | H | CH₃ |
| 61. | 4-CH₃-phényl | (CH₂) ₂ | H | 2 | 2 | H | H |
| 62. | 4-CH₃-phényl | (CH₂)₂ | H | 2 | 2 | H | CH₃ |
| 63. | 4-CH₃O-phényl | (CH₂)₂ | H | 2 | 2 | H | H |
| 64. | 9-CH₃O-phényl | (CH₂) ₂ | H | 2 | 2 | H | CH₃ |
| 65. | 2-phényl-phényl | (CH₂)₂ | H | 2 | 2 | H | CH₃ |
| 66. | 3-phényl-phényl | (CH₂)₂ | H | 2 | 2 | H | CH₃ |
| 67. | naphtalèn-1-yl | (CH₂) ₂ | H | 2 | 2 | H | CH₃ |
| 68. | naphtalèn-2-yl | (CH₂)₂ | H | 2 | 2 | H | CH₃ |
| 69. | pyrimidin-2-yl | (CH₂) ₂ | H | 2 | 2 | H | CH₃ |
| 70. | pyrimidin-5-yl | (CH₂) ₂ | H | 2 | 2 | H | CH₃ |
| 71. | 6-cyclopentyl-pyridin-2-yl | (CH₂) ₂ | H | 2 | 2 | H | CH₃ |
| 72. | 6-pyrrolidin-1-ylpyridin-2-yl | (CH₂) ₂ | H | 2 | 2 | H | CH₃ |
| 73. | thiazol-2-yl | (CH₂) ₂ | H | 2 | 2 | H | CH₃ |
| 74. | isoquinolin-1-yl | (CH₂) ₂ | H | 2 | 2 | H | CH₃ |
| 75. | 1,2,3,4-tétrahydroquinolin-1-yl | (CH₂)₂ | H | 2 | 2 | H | H |
| 76. | 1,2,3,4-tétrahydroisoquinolin -2-yl | (CH₂) ₂ | H | 2 | 2 | H | H |
| 77. | indol-1-yl | (CH₂) ₂ | H | 2 | 2 | H | H |
| 78. | indolin-1-yl | (CH₂)₂ | H | 2 | 2 | H | H |
| 79. | pyrrole[2,3-b] pyridin-1-yl | (CH₂)₂ | H | 2 | 2 | H | H |
| 80. | benzimidazol-1-yl | (CH₂)₂ | H | 2 | 2 | H | H |
| 81. | 4-phénylimidazol-1-yl | (CH₂)₂ | H | 2 | 2 | H | H |
| 82. | 3-C1-phényl | (CH₂) ₃ | H | 2 | 2 | H | CH₃ |
| 83. | 4-Cl-phényl | (CH₂) ₃ | H | 2 | 2 | H | CH₃ |
| 84. | 3-CF₃-phényl | (CH₂) ₃ | H | 2 | 2 | H | CH₃ |
| 85. | 4-CF₃-phényl | (CH₂) ₃ | H | 2 | 2 | H | CH₃ |
| 86. | 3-CN-phényl | (CH₂)₃ | H | 2 | 2 | H | CH₃ |
| 87. | 2-phényl-phényl | (CH₂)₃ | H | 2 | 2 | H | CH₃ |
| 88. | 3-phényl-phényl | ((CH₂)₃ | H | 2 | 2 | H | CH₃ |
| 89. | napthalèn-1-yl | (CH₂)₃ | H | 2 | 2 | H | CH₃ |
| 90. | napthalèn-2-yl | (CH₂)₃ | H | 2 | 2 | H | CH₃ |
| 91. | pyrimidin-2-yl | (CH₂)₃ | H | 2 | 2 | H | CH₃ |
| 92. | pyrimidin-5-yl | (CH₂)₃ | H | 2 | 2 | H | CH₃ |
| 93. | thiazol-2-yl | (CH₂) ₃ | H | 2 | 2 | H | CH₃ |
| 94. | 3-Cl-phényl | C≡C | H | 2 | 2 | H | CH₃ |
| 95. | 4-Cl-phényl | C≡C | H | 2 | 2 | H | CH₃ |
| 96. | 3-CF₃-phényl | C≡C | H | 2 | 2 | H | CH₃ |
| 97. | 9-CF₃-phényl | C≡C | H | 2 | 2 | H | CH₃ |
| 98. | 3-CN-phényl | C≡C | H | 2 | 2 | H | CH₃ |
| 99. | 2-phényl-phényl | C≡C | H | 2 | 2 | H | CH₃ |
| 100 | 3-phényl-phényl | C≡C | H | 2 | 2 | H | CH₃ |
| 101 | naphtalèn-1-yl | C≡C | H | 2 | 2 | H | CH₃ |
| 102 | naphtalèn-2-yl | C≡C | H | 2 | 2 | H | CH₃ |
| 103 | pyrimidin-2-yl | C≡C | H | 2 | 2 | H | CH₃ |
| 104 | pyrimidin-5-yl | C≡C | H | 2 | 2 | H | CH₃ |
| 105 | thiazol-2-yl | C≡C | H | 2 | 2 | H | CH₃ |
| 106 | 3-Cl-phényl | C≡CCH₂ | H | 2 | 2 | H | CH₃ |
| 107 | 4-Cl-phényl | C≡CCH₂ | H | 2 | 2 | H | CH₃ |
| 108 | 3-CF₃-phényl | C≡CCH₂ | H | 2 | 2 | H | CH₃ |
| 109 | 4-CF₃-phényl | C≡CCH₂ | H | 2 | 2 | H | CH₃ |
| 110 | 3-CN-phényl | C≡CCH₂ | H | 2 | 2 | H | CH₃ |
| 111 | 2-phényl-phényl | C≡CCH₂ | H | 2 | 2 | H | CH₃ |
| 112 | 3-phényl-phényl | C≡CCH₂ | H | 2 | 2 | H | CH₃ |
| 113 | naphtalèn-1-yl | C≡CCH₂ | H | 2 | 2 | H | CH₃ |
| 114 | naphtalèn-2-yl | C≡CCH₂ | H | 2 | 2 | H | CH₃ |
| 115 | pyrimidin-2-yl | C≡CCH₂ | H | 2 | 2 | H | CH₃ |
| 116 | pyrimidin-5-yl | C≡CCH₂ | H | 2 | 2 | H | CH₃ |
| 117 | thiazol-2-yl | C≡CCH₂ | H | 2 | 2 | H | CH₃ |
| 118 | 6-pyrrolidin-1-ylpyridin-2-yl | CH₂ | H | 2 | 2 | H | CH₃ |
| 119 | 6-(1-isopropylpiperidin-4-yl)pyridin-2-yl | (CH₂) ₂ | H | 2 | 2 | H | CH₃ |

Dans le cadre de l'invention, les composés de formule générale (I) peuvent donc comporter plusieurs groupes A identiques ou différents entre eux.

Parmi les composés selon l'invention, les composés suivants peuvent être citées :
- 4-{5-[4-(trifluorométhyl)phényl]pyridin-2-yl}pipéridine-1-carboxylate de 2-(méthylamino)-2-oxoéthyle
- 4-(4'-chlorobiphényl-4-yl)-4-hydroxypipéridine-1-carboxylate de 2-(méthylamino)-2-oxoéthyle
- 4-(4'-éthoxybiphényl-4-yl)-4-hydroxypipéridine-1-carboxylate de 2-(méthylamino)-2-oxoéthyle
- 4-(3',4'-dichlorobiphényl-4-yl)-4-hydroxypipéridine-1-carboxylate de 2-(méthylamino)-2-oxoéthyle
- 4-(3'-chloro-4'-fluorobiphényl-4-yl)-4-hydroxypipéridine-1-carboxylate de 2-(méthylamino)-2-oxoéthyle
- 4-[(6-cyclopentylpyridin-2-yl)méthyl]pipéridine-1-carboxylate de 2-(méthylamino)-2-oxoéthyle
- 4-[2-(3-chlorophényl)éthyl]pipéridine-1-carboxylate de 2-(méthylamino)-2-oxoéthyle
- 4-[2-(4-chlorophényl)éthyl]pipéridine-1-carboxylate de 2-(méthylamino)-2-oxoéthyle
- 4-{2-[3-(trifluorométhyl)phényl]éthyl}pipéridine-1-carboxylate de 2-(méthylamino)-2-oxoéthyle
- 4-{2-[4-(trifluorométhyl)phényl]éthyl}pipéridine-1-carboxylate de 2-(méthylamino)-2-oxoéthyle
- 4-(2-biphényl-3-yléthyl)pipéridine-1-carboxylate de 2-(méthylamino)-2-oxoéthyle
- 4-[2-(1-naphtyl)éthyl]pipéridine-1-carboxylate de 2-(méthylamino)-2-oxoéthyle
- 4-[2-(2-naphtyl)éthyl]pipéridine-1-carboxylate de 2-(méthylamino)-2-oxoéthyle
- 4-[2-(6-cyclopentylpyridin-2-yl)éthyl]pipéridine-1-carboxylate de 2-(méthylamino)-2-oxoéthyle
- 4-[2-(6-pyrrolidin-1-ylpyridin-2-yl)éthyl]pipéridine-1-carboxylate de 2-(méthylamino)-2-oxoéthyle
- 4-(2-isoquinolin-1-yléthyl)pipéridine-1-carboxylate de 2-(méthylamino)-2-oxoéthyle
- 4-[3-(3-chlorophényl)propyl]pipéridine-1-carboxylate de 2-(méthylamino)-2-oxoéthyle
- 4-[3-(4-chlorophényl)propyl]pipéridine-1-carboxylate de 2-(méthylamino)-2-oxoéthyle
- 4-{3-[3-(trifluorométhyl)phényl]propyl}pipéridine-1-carboxylate de 2-(méthylamino)-2-oxoéthyle
- 4-{3-[4-(trifluorométhyl)phenyl]propyl}pipéridine-1-carboxylate de 2-(méthylamino)-2-oxoéthyle
- 4-[3-(3-cyanophényl)propyl]pipéridine-1-carboxylate de 2-(méthylamino)-2-oxoéthyle
- 4-(3-biphényl-2-ylpropyl)pipéridine-1-carboxylate de 2-(méthylamino)-2-oxoéthyle
- 4-(3-biphényl-3-ylpropyl)pipéridine-1-carboxylate de 2-(méthylamino)-2-oxoéthyle
- 4-[3-(1-naphtyl)propyl]pipéridine-1-carboxylate de 2-(méthylamino)-2-oxoéthyle
- 4-[3-(2-naphtyl)propyl]pipéridine-1-carboxylate de 2-(méthylamino)-2-oxoéthyle
- 4-[3-(1,3-thiazol-2-yl)propyl]pipéridine-1-carboxylate de 2-(méthylamino)-2-oxoéthyle
- 4-[(3-chlorophényl)éthynyl]pipéridine-1-carboxylate de 2-(méthylamino)-2-oxoéthyle
- 4-[(4-chlorophényl)éthynyl]pipéridine-1-carboxylate de 2-(méthylamino)-2-oxoéthyle
- 4-(biphényl-3-yléthynyl)pipéridine-1-carboxylate de 2-(méthylamino)-2-oxoéthyle
- 4-(1-naphtyléthynyl)pipéridine-1-carboxylate de 2-(méthylamino)-2-oxoéthyle
- 4-(2-naphtyléthynyl)pipéridine-1-carboxylate de 2-(méthylamino)-2-oxoéthyle
- 4-(3-biphényl-2-ylprop-2-yn-1-yl)pipéridine-1-carboxylate de 2-(méthylamino)-2-oxoéthyle
- 4-[(6-pyrrolidin-1-ylpyridin-2-yl)méthyl]pipéridine-1-carboxylate de 2-(méthylamino)-2-oxoéthyle.

Parmi les composés selon l'invention les composés suivants peuvent être cités :
- 4-phénylpipéridine-1-carboxylate dé 2-amino-2-oxoéthyle ;
- 4-phénylpipéridine-1-carboxylate de 2-(méthylamino)-2-oxoéthyle ;
- 4-[3-(trifluorométhyl)phényl]pipéridine-1-carboxylate de 2-amino-2-oxoéthyle ;
- 4-[3-(trifluorométhyl)phényl]pipéridine-1-carboxylate de 2-(méthylamino)-2-oxoéthyle ;
- 4-(4-phényl-1H-imidazol-1-yl)pipéridine-1-carboxylate de 2-(méthylamino)-2-oxoéthyle :
- 9-(1H-1,2,3-benzotriazol-1-yl)pipéridine-1-carboxylate de 2-(méthylamino)-2-oxoéthyle ;
- 4-(4-bromophényl)-4-hydroxypipéridine-1-carboxylate de 2-(méthylamino)-2-oxoéthyle ;
- 4-(4'-fluorobiphényl-4-yl)-4-hydroxypipéridine-1-carboxylate de 2-(méthylamino)-2-oxoéthyle ;
- 4-(4'-chlorobiphényl-4-yl)-4-hydroxypipéridine-1-carboxylate de 2-(méthylamino)-2-oxoéthyle ;
- 4-hydroxy-4-(4'-méthylbiphényl-4-yl)pipéridine-1-carboxylate de 2-(méthylamino)-2-oxoéthyle ;
- 4-(4'-butylbiphényl-4-yl)-4-hydroxypipéridine-1-carboxylate de 2-(méthylamino)-2-oxoéthyle ;
- 4-hydroxy-4-[4'-(trifluorométhyl)biphényl-4-yl]pipéridine-1-carboxylate de 2-(méthylamino)-2-oxoéthyle ;
- 4-hydroxy-4-[4'-(méthyloxy)biphényl-4-yl]pipéridine-1-carboxylate de 2-(méthylamino)-2-oxoéthyle :
- 4-[4'-(éthyloxy)biphényl-4-yl]-4-hydroxypipéridine-1-carboxylate de 2-(méthylamino)-2-oxoéthyle ;
- 4-(3',4'-dichlorobiphényl-4-yl)-4-hydroxypipéridine-1-carboxylate de 2-(méthylamino)-2-oxoéthyle ;
- 4-[3'-fluoro-4'-(méthyloxy)biphényl-4-yl]-4-hydroxypipéridine-1-carboxylate de 2-(méthylamino)-2-oxoéthyle ;
- 4-(3'-chloro-4'-fluorobiphényl-4-yl)-4-hydroxypipéridine-1-carboxylate de 2-(méthylamino)-2-oxoéthyle ;
- 4-(naphthalèn-2-ylméthyl)pipéridine-1-carboxylate de 2-(méthylamino)-2-oxoéthyle ;
- 4-(biphényl-4-ylméthyl)pipéridine-1-carboxyl de 2-(méthylamino)-2-oxoéthyle ;
- 4-(1H-indol-1-ylméthyl)pipéridine-1-carboxylate de 2-amino-2-oxoéthyle ;
- 4-(2,3-dihydro-1H-indol-1-ylméthyl)pipéridine-1-carboxylate de 2-amino-2-oxoéthyle ;
- 4-(3,4-dihydroquinolin-1(2H)-ylméthyl)pipéridine-1-carboxylate de 2-amino-2-oxoéthyle ;
- 4-(3,4-dihydroisoquinolin-2(1H)-ylméthyl)pipéridine-1-carboxylate de 2-amino-2-oxoéthyle ;
- 4-(1H-pyrrolo[2,3-b]pyridin-1-ylméthyl)pipéridine-1-carboxylate de 2-amino-2-oxoéthyle ;
- 4-(1H-benzimidazol-1-ylméthyl)pipéridine-1-carboxylate de 2-amino-2-oxoéthyle ;
- 4-[(4-phényl-1H-imidazol-1-yl)méthyl]pipéridine-1-carboxylate de 2-amino-2-oxoéthyle ;
- 3-(2-phényléthyl)pyrrolidine-1-carboxylate de 2-amino-2-oxoéthyle ;
- 4-[2-(3,4-dihydroquinolin-1(2H)-yl)éthyl]pipéridine-1-carboxylate de 2-amino-2-oxoéthyle ;
- 4-[2-(3,4-dihydroisoquinolin-2(1H)-yl)éthyl]pipéridine-1-carboxylate de 2-amino-2-oxoéthyle ;
- 4-[2-(1H-indol-1-yl)éthyl]pipéridine-1-carboxylate de 2-amino-2-oxoéthyle ;
- 4-[2-(2,3-dihydro-1H-indol-1-yl)éthyl]pipéridine-1-carboxylate de 2-amino-2-oxoéthyle ;
- 4-[2-(1H-pyrrolo[2,3-b]pyridin-1-yl)éthyl]pipéridine-1-carboxylate de 2-amino-2-oxoéthyle ;
- 4-[2-(1H-benzimidazol-1-yl)éthyl]pipéridine-1-carboxylate de 2-amino-2-oxoéthyle ;
- 4-[2-(4-phényl-1H-imidazol-1-yl)éthyl]pipéridine-1-carboxylate de 2-amino-2-oxoéthyle.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention. Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, tertbutyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcènylène, un groupe aliphatique à 2 carbones, insaturé divalent, plus particulièrement un éthylène
- C₂-alcynylène, un groupe -C≡C- ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- fluoroalkyle, un groupe alkyle dont un ou plusieurs atomes d'hydrogène ont été substitués par un atome de fluor ;
- fluoroalcoxy, un groupe alcoxy dont un ou plusieurs atomes d'hydrogène ont été substitués par un atome de fluor ;
- fluorothioalkyle, un groupe thioalkyle dont un ou plusieurs atomes d'hydrogène ont été substitués par un atome de fluor
- atome d'halogène, un fluor, un chlore, un brome ou un iode.

Les composés de l'invention peuvent être préparés selon la méthode illustrée par le schéma qui suit.

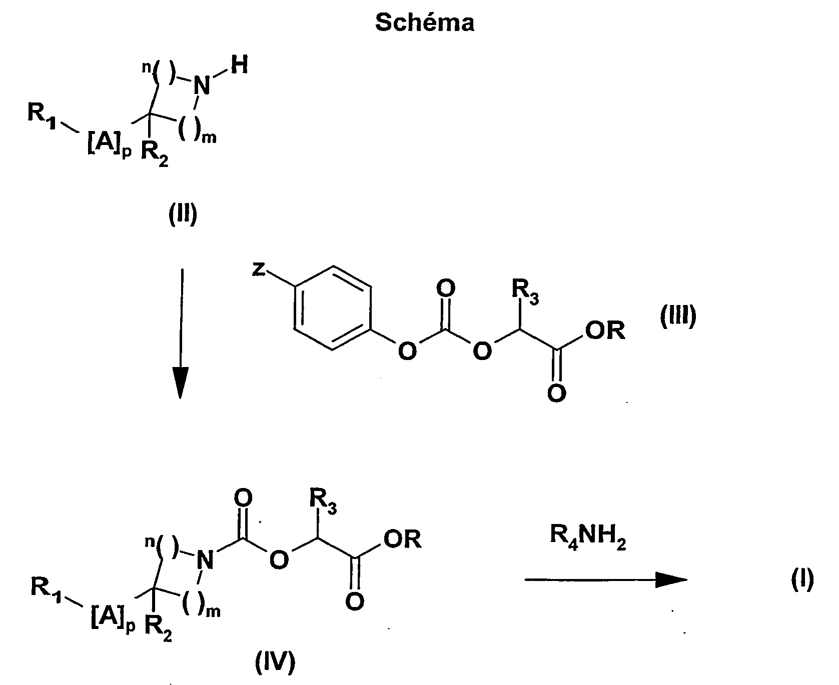

Les composés de l'invention peuvent être préparés en faisant réagir une amine de formule générale (II), dans laquelle R₁, A, R₂, p, m et n sont tels que définis dans la formule générale (I), avec un carbonate de formule générale (III), dans laquelle Z représente un atome d'hydrogène ou un groupement nitro, R₃ est tel que défini dans la formule générale (I) et R représente un groupe méthyle ou éthyle, dans un solvant tel que le toluène, le dichloroéthane, l'acétonitrile, ou un mélange de ces solvants, à une température comprise entre 0°C et 80°C. Les carbamate-esters de formule générale (IV) ainsi obtenus sont ensuite transformés en composés de formule générale (I) par aminolyse au moyen d'une amine de formule générale R₄NH₂ où R₄ est tel que défini dans la formule générale (I). La réaction d'aminolyse peut être réalisée dans un solvant tel que le méthanol ou l'éthanol, ou un mélange de solvants tels que le méthanol et le tétrahydrofurane.

Les composés de formule générale (I) ou (IV), dans laquelle R₁ représente un groupe de type aryle-aryle, aryle-hétéroaryle, hétéroaryle-aryle ou hétéroaryle-hétéroaryle, peuvent être également préparés par réaction des composés de formule générale (I) ou (IV) correspondants, pour lesquels R₅ est substitué par un atome de chlore, de brome, d'iode ou par un groupement triflate dans la position où le groupe R₇ doit être introduit, avec un dérivé d'acide aryl- ou hétéroaryl-boronique suivant les conditions de réaction de Suzuki (Chem. Rev. 1995, 95, 2457-2483) ou avec un dérivé d'aryl- ou d'hétéroaryl-tri-alkylstannane suivant les conditions de réaction de Stille (Angew. Chem. Int. Ed. 1986, 25, 504-524).

Les carbonates de formule générale (III) peuvent être préparés selon toute méthode décrite dans la littérature, par exemple par réaction d'un alcool de formule générale HOCHR₃COOR où R représente un groupe méthyle ou éthyle, avec le chloroformiate de phényle ou de 4-nitrophényle, en présence d'une base telle que la triéthylamine ou la diisopropyléthylamine.

Les composés de formule générale (II) ainsi que les amines de formule générale R₄NH₂, quand leur mode de préparation n'est pas décrit, sont disponibles dans le commerce ou décrits dans la littérature, ou peuvent être préparés selon différentes méthodes décrites dans la littérature ou connues de l'homme du métier.

Les composés de formule générale (IV), dans laquelle R₁, A, R₂, R₃, p, m et n sont tels que définis dans la formule générale (I) et R représente un groupe méthyle ou éthyle, sont nouveaux et font également partie de l'invention. Ils sont utiles en tant qu'intermédiaires de synthèse pour la préparation des composés de formule générale (I).

Les exemples qui vont suivre illustrent la préparation de quelques composés de l'invention. Les microanalyses, les spectres I.R. et R.M.N. et/ou la LC-MS (Liquid Chromatography coupled to Mass Spectroscopy) confirment les structures et les puretés des composés obtenus.

PF(°C) représente le point de fusion en degrés Celsius. Les numéros indiqués entre parenthèses dans les titres des exemples correspondent à ceux de la 1ère colonne du tableau ci-après.

La nomenclature UICPA (Union Internationale de Chimie Pure et Appliquée - IUPAC en anglais) a été utilisée pour la dénomination des composés dans les exemples suivants. Par exemple, pour le groupe biphényle, la numérotation suivante a été respectée :

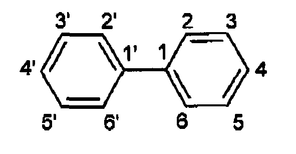

### Exemple 1 (composé n° 14)

### 4-(4-phényl-1H-imïdazol-1-y1)pipéridine-1-oarboxylate de 2-(màthylamino)-2-oxoéthyle

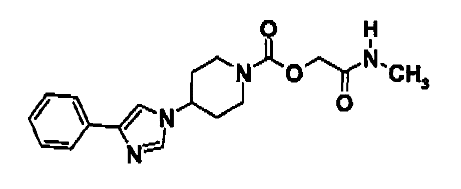

### 1.1. 4-[(méthylsulfonyl)oxy]pipéridine-1-carboxylate de 1,1-diméthyléthyle

Sous agitation, on ajoute goutte à goutte 1,4 ml (17,9 mmoles) de chlorure de méthanesulfonyle à une solution de 3,0 g (14,9 mmoles) de 4-hydroxypipéridine-1-carboxylate de 1,1-diméthyléthyle et de 2,2 ml (17,9 mmoles) de triéthylamine dans 60 ml de dichlorométhane refroidie par un bain de glace. On poursuit l'agitation pendant une heure à 0°C puis 4 heures à température ambiante. On dilue le mélange réactionnel par 100 ml de dichlorométhane et on lave successivement par 100 ml d'une solution aqueuse d'hydrogénocarbonate de sodium, puis par une solution aqueuse saturée en chlorure d'ammonium et une solution aqueuse saturée en chlorure de sodium. On sèche sur sulfate de sodium et on évapore à sec. On triture ensuite le résidu dans un mélange 50/50 de cyclohexane et de diéthyléther pour obtenir 3,7 g de produit sous forme de solide blanc.

### 1.2. 4-(4-phényl-1H-imidazol-1-yl)pipéridine-1-carboxylate de 1,1-diméthyléthyle

A une suspension de 1,1 g (27,9 mmoles) d'hydrure de sodium (suspension à 60% dans l'huile) dans 30 ml de *N,N-*diméthylformamide refroidie par un bain de glace, on ajoute goutte à goutte une solution de 4,0 g (27,9 mmoles) de 4-phénylimidazole dans 40 ml de *N*,*N*-diméthylformamide. On agite ensuite une heure à température ambiante puis on refroidit à 0°C et on ajoute goutte à goutte 2,6 g (9,3 mmoles) de 4-[(méthylsulfonyl)oxy]pipéridine-1-carboxylate de 1,1-diméthyléthyle obtenu à l'étape 1.1., en solution dans 20 ml de *N*,*N*-diméthylformamide. On chauffe ensuite à 80°C pendant 2 heures. On refroidit le mélange réactionnel à température ambiante et on le dilue par 150 ml 'd'eau et 150 ml d'acétate d'éthyle. On décante et on extrait deux fois la phase aqueuse par 100 ml d'acétate d'éthyle. On lave les phases organiques par deux fois 100 ml d'eau puis par 100 ml d'une solution aqueuse saturée en chlorure de sodium. On sèche sur sulfate de sodium et on évapore à sec. On purifie le résidu par chromatographie sur gel de silice en éluant par un mélange 98/2 de dichlorométhane et de méthanol pour obtenir 1,0 g de produit sous forme d'huile jaune.

### 1.3. 4-(4-phényl-1H-imidazol-1-yl)pipéridine

On ajoute goutte à goutte 5,6 ml (76,3 mmoles) d'acide trifluoroacétique à une solution de 1,0 g (3,05 mmoles) de 4-(4-phényl-1*H*-imidazol-1-yl)pipéridine-1-carboxylate de 1,1-diméthyléthyle obtenu à l'étape 1.2., dans 60 ml de dichlorométhane refroidie par un bain de glace. On agite ensuite une heure à température ambiante et on évapore à sec. On reprend le résidu dans 25 ml d'eau et on ajoute 2 ml d'une solution aqueuse à 30% d'hydroxyde de sodium. On agite pendant 30 minutes puis on extrait quatre fois par 80 ml de dichlorométhane. On lave ensuite les phases organiques par une solution aqueuse saturée en chlorure de sodium, on les sèche sur sulfate de sodium et on les évapore à sec pour obtenir 0,7 g de produit sous forme d'huile jaune, utilisé tel quel dans l'étape suivante.

### 1.4. 4-(4-phényl-1H-imidazol-1-yl)pipéridine-1-carboxylate de 2-(éthyloxy)-2-oxoéthyle

On chauffe à 60°C pendant une nuit, une solution de 1,0 g (4,4 mmoles) de 4-(4-phényl-1*H*-imidazol-1-yl)pipéridine préparé selon l'étape 1.3. et de 1,18 g (5,2 mmoles) de [(phënyloxycarbonyl)oxy]acétate d'éthyle (J. Med. Chem, 1999, 42, 277-290) dans 50 ml de toluène. On évapore ensuite à sec et on reprend le résidu par 80 ml d'acétate d'éthyle et 80 ml d'eau. On décante et on extrait la phase aqueuse par trois fois 80 ml d'acétate d'éthyle. On lave ensuite les phases organiques par 80 ml d'une solution aqueuse saturée en chlorure de sodium. On les sèche sur sulfate de sodium et on évapore à sec. On purifie le résidu par chromatographie sur gel de silice en éluant par un mélange 98/2 de dichlorométhane et de méthanol pour obtenir 0,35 g de produit.

### 1.5. 4-(4-phényl-1H-imidazol-1-yl)pipéridine-1-carboxylate de 2-(méthylamino)-2-oxoéthyle

On dissout 0,35 g (0,98 mmole) de 4-(4-phényl-1*H*-imidazol-1-yl)pipéridine-1-carboxylate de 2-(éthyloxy)-2-oxoéthyle obtenu à l'étape 1.4., dans 7 ml de méthanol. On ajoute 1,5 ml (3 mmoles) d'une solution 2M de méthylamine dans le tétrahydrofurane. Après 16 heures à température ambiante, on rajoute 1 ml (2 mmoles) d'une solution 2M de méthylamine dans le tétrahydrofurane et on laisse réagir 6 heures supplémentaires. On évapore à sec et on purifie le résidu par chromatographie sur gel de silice en éluant par un mélange 98/2 puis 97/3, 96/4 et 95/5 de dichlorométhane et de méthanol. On triture ensuite dans du diéthyléther pour obtenir 0,20 g de produit sous forme de solide blanc.
Point de fusion (°C) : 192-194
LC-MS : M+H = 343
RMN-¹H (CDCl₃) δ (ppm) : 7, 75 (d, 2H); 7,60 (s, 1H) ; 7,40 (m, 2H); 7,25 (m, 2H); 6,05 (s large, 1H) ; 4,65 (s, 2H); 4,35 (m, 2H); 4,15 (m, 1H); 3,05 (m, 2H); 2,90 (d, 3H); 2,20 (m, 2H); 2,05-1,85 (m, 2H).

### Exemple 2 (composé n° 32)

### 4-(4-bromophényl)-4-hydroxypipéridine-1-carboxylate de 2-(méthylamino)-2-oxoéthyle

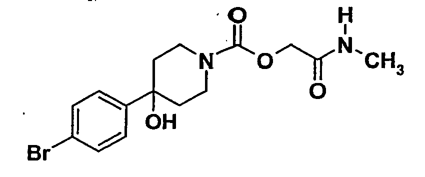

### 2.1. 4-(4-bromophényl)-4-hydroxypipéridine-1-carboxylate de 2-(éthyloxy)-2-oxyéthyle

On chauffe à 50°C pendant 20 heures un mélange de 2,24 g (10 mmoles) de [(phényloxycarbonyl)oxy]acétate d'éthyle et 2,56 g (10 mmoles) de 4-(4-bromophényl)-4-pipéridinol en solution dans 40 ml de toluène. On évapore à sec la solution au bain-marie sous pression réduite. On obtient une huile qui est directement utilisée dans l'étape suivante.

### 2.2. 4-(4-bromophényl)-4-hydroxypipéridine-1-carboxylate de 2-(méthylamino)-2-oxoéthyle

Le 4-(4-bromophényl)-4-hydroxypipéridine-1-carboxylate de 2-(éthyloxy)-2-oxyéthyle obtenu à l'étape 2.1., est agité pendant 3 heures dans une solution de méthylamine à 33% dans le méthanol. On concentre la solution au bain-marie sous pression réduite. On purifie le résidu par chromatographie sur gel de silice en éluant à l'acétate d'éthyle. On obtient 2,6 g de produit sous forme d'huile qui se solidifie progressivement.
Point de fusion (°C) :57-60
LC-MS : M+H = 371
RMN-¹H (DMSO-d₆) δ (ppm) : 7, 55 (s large, 1H) ; 7, 50 (d, 2H) ; 7,40(d, 2H); 5,20(s, 1H) ; 4,40(s, 2H); 3, 80 (m, 2H); 3,20(m, 2H); 2,60(d, 3H); 1,90-1,50(m, 4H).

### Exemple 3 (composé n° 40)

### 4-(3',4'-dichlorobiphényl-4-yl)-4-hydroxypipéridine-1-carboxylate de 2-(méthylamino)-2-oxoéthyle

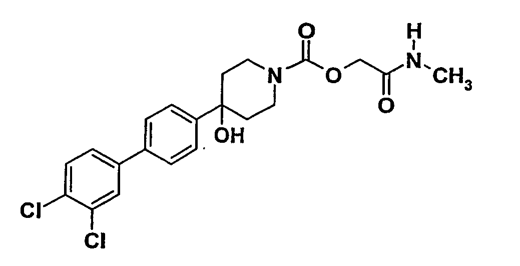

On mélange 0,1 g (0,27 mmole) de 4-(4-bromophényl)-4-hydroxypipéridine-1-carboxylate de 2-(méthylamino)-2-oxoéthyle obtenu suivant l'exemple 2, 0,077 g (0,4 mmole) d'acide 3,4-dichlorophénylboronique, 10 mg de tétrakis-(triphënylphosphine)palladium(0), 2 ml de solution aqueuse 2M de carbonate de sodium, 0,5 ml d'éthanol et 4 ml de toluène préalablement dégazé à l'azote. On chauffe à 80°C sous agitation pendant 20 heures. On filtre à chaud sur une cartouche hydrophobe, on rince avec du tétrahydrofurane (THF) et on évapore à sec. On purifie lé résidu par chromatographie LC-MS sur phase de silice en éluant avec un gradient de cyclohexane / acétate d'éthyle / méthanol pour obtenir 0,069 g de produit cristallin.
Point de fusion (°C) : 156-158
LC-MS : M+H = 438
RMN-¹H (DMSO-d₆) δ (ppm) : 7,95 (s, 1H); 7,80 (m, 1H); 7,70 (m, 4H); 7,60 (m, 2H); 5,20 (s, 1H); 4,45 (s, 2H); 4,00 (m, 2H); 3,25 (m, 2H); 2,60 (d, 3H); 1,95 (m, 2H); 1,65 (m, 2H) .

### Exemple 4 (composé n° 43)

### 4-(naphtalèn-2-ylméthyl)pipéridine-1-carboxylate de 2-(méthylamino)-2-oxoéthyle

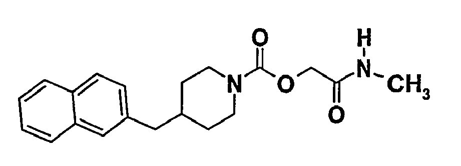

### 4.1. 4-(naphtalèn-2-ylméthyl)pipéridine-1-carboxylate de 1,1-diméthyléthyle

Sous atmosphère d'argon, on ajoute 8,0 ml d'une solution 0,5 N (4 mmoles) de 9-borabicyclo[3.3.1]nonane dans le tétrahydrofurane à une solution de 0,789 g (4 mmoles) de 4-méthylidènepipéridine-1-carboxylate de 1,1-diméthyléthyle (Tetrahedron Letters 1996, 37(30), 5233-5234) en solution dans 5 ml de tétrahydrofurane. On chauffe à reflux pendant 3 heures. On refroidit à température ambiante et on ajoute 0,787 g (3,8 mmoles) de 2-bromonaphtalène en solution dans 9 ml de *N*,*N*-diméthylformamide, 0,829 g (6,0 mmoles) de carbonate de potassium en solution dans 1 ml d'eau et 0,16 g (0,20 mmole) du complexe [1,1'-bis(diphénylphosphine)-ferrocene]dichloropalladium(II)-dichlorométhane). On chauffe à reflux pendant une nuit. On dilue le mélange réactionnel par 150 ml d'acétate d'éthyle et 50 ml d'eau. On décante la phase organique et on la lave par 25 ml d'eau puis par 25 ml d'une solution aqueuse saturée en chlorure de sodium. On la sèche sur sulfate de magnésium et on l'évapore sous vide. On purifie le résidu par chromatographie sur gel de silice en éluant par un mélange 99/1 puis 95/5 et 90/10 de cycLohexane et d'acétate d'éthyle pour obtenir 0,79 g de produit sous forme de liquide visqueux incolore.

### 4.2. 4-(naphtalèn-2-ylméthyl)pipéridine

On dissout 0,79 g (2,43 mmoles) de 4-(naphtalèn-2-ylméthyl)pipéridine-1-carboxylate de 1,1-diméthyléthyle obtenu à l'étape 4.1., dans 10 ml de dichlorométhane et on ajoute 2 ml (25 mmoles) d'acide trifluoroacétique. On agite pendant 3 heures à température ambiante. On évapore sous pression réduite puis on ajoute 4 ml de 1,2-dichloroéthane et on évapore à nouveau. On reprend le résidu dans un mélange de 50 ml de dichlorométhane et de 15 ml d'une solution aqueuse à 10% d'hydroxyde de sodium. On décante la phase organique et on extrait deux fois la phase aqueuse par 25 ml de dichlorométhane. Les phases organiques ont lavées par 15 ml d'une aqueuse saturée en chlorure de sodium puis séchées sur sulfate de sodium et évaporées sous vide pour fournir 0,52 g de produit sous forme d'huile orange, utilisé tel quel dans l'étape suivante.

### 4.3. 4-(naphtalèn-2-ylméthyl)pipéridine-1-carboxylate de 2-(éthoxy)-2-oxoéthyle

On chauffe à 60°C pendant une nuit, un mélange de 0,52 g (2,3 mmoles) de 4-(naphtalèn-2-ylméthyl)pipéridine obtenu à l'étape 4.2., et de 0,69 g (3,11 mmoles) de [(phényloxycarbonyl)oxy]acétate d'éthyle dans 10 ml de toluène et 5 ml d'acétonitrile. On évapore sous vide. On purifie le résidu par chromatographie sur gel de silice en éluant par un mélange 90/10 puis 85/15 et 80/20 de cyclohexane et d'acétate d'éthyle pour obtenir 0,56 g de produit sous forme de liquide visqueux incolore.

### 4.4. 4-(naphtalèn-2-ylméthyl)pipéridine-1-carboxylate de 2-(méthylamino)-2-oxoéthyle

On dissout dans 3 ml de méthanol 0,54 g (1,52 mmole) de 4-(naphtalèn-2-ylméthyl)pipéridine-1-carboxylate de 2-(éthoxy)-2-oxoéthyle obtenu à l'étape 4.3. et on ajoute 3 ml (6,0 mmoles) d'une solution de méthylamine 2 M dans le tétrahydrofurane. On laisse réagir une nuit à température ambiante puis on ajoute 1,5 g de silice et on évapore. On purifie le résidu par chromatographie sur gel de silice en éluant par un mélange 98,5/1,5 puis 97/3 de dichlorométhane et de méthanol. On recristallise ensuite dans un mélange d'acétate d'éthyle et de diisopropyléther pour obtenir 0,43 g de produit sous forme de solide blanc.
Point de fusion (°C) : 150-152
LC-MS : M+H 341
RMN-1H (CDCl₃) δ (ppm) : 7,80 (m, 3H); 7,60 (s, 1H) ; 7,45 (m, 2H); 7,30 (d, 1H); 6,10 (m, 1H) ; 4,60 (s, 2H); 4, 15 (m, 2H); 2,85 (d, 3H); 2,85-2,75 (m+d, 4H); 1,90-1,70 (m, 3H); 1,35-1,15 (m, 2H).

### Exemple 5 (composé n°107)

### 4-[3-(4-chlorophényl)prop-2-yn-1-yl]pipéridine-1-carboxylate de 2-(méthylamino)-2-oxoéthyle

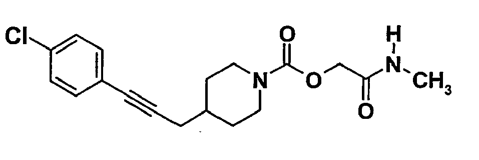

### 5.1. 4-(2-oxoéthyl)pipéridine-1-carboxylate de tert-butyle

A une solution 30,4 g (132 mmoles) de 4-(2-hydroxyéthyl)pipéridine-1-carboxylate de *tert*-butyle dans 150 mL de dichlorométhane, refroidie à 0°C, on ajoute par portion 70,9 g (167 mmoles) de (1,1,1-tris (acétyloxy)-1,1-dihydro-1,2-benziodoxol-3-(1*H*)-one (réactif de Dess-Martin). On agite 2 heures à température ambiante puis on ajoute 150 mL d'une solution aqueuse à 10% de thiosulfate de sodium (Na₂S₂O₃) et on poursuit l'agitation pendant 30 minutes supplémentaires. On décante la phase organique, on la lave par une solution aqueuse saturée en carbonate de sodium, on la sèche sur sulfate de sodium et on l'évapore à sec pour obtenir 30,1 g (132 mmoles) de produit sous forme d'huile incolore utilisé tel quel dans l'étape suivante.

### 5.2. 4-(3,3-dibromoprop-2-en-1-yl)pipéridine-1-carboxylate de tert-butyle

A une solution de 139,4 g (531 mmoles)de triphénylphosphine dans 440 mL de toluène, refroidie à -20°C, on ajoute 47,6 mL (531 mmoles) de tribromométhane puis 59,6 g (531 mmoles) de tert-butylate de potassium. On poursuit l'agitation à -20°C pendant 15 minutes puis on ajoute une solution de 30,1 g (131 mmoles) de 4-(2-oxoéthyl)pipéridine-1-carboxylate de tert-butyle, préparé à l'étape 5.1., dans 240 mL de toluène. On agite ensuite à température ambiante pendant 3 heures. On ajoute 300 mL de diéthyléther, on filtre le solide formé et on évapore le filtrat. On purifie le résidu par chromatographie sur gel de silice en éluant par du dichlorométhane pour obtenir 32,6 g (85 mmoles) de produit sous forme d'huile jaune.

### 5.3. 4-prop-2-yn-1-ylpipéridine-1-carboxylate de tert-butyle

On dissout 32,6 g (85 mmoles) de 4-(3,3-dibromoprop-2-en-1-yl)pipéridine-1-carboxylate de tert-butyle, préparé à l'étape 5.2., dans 420 mL de tétrahydrofurane anhydre. On refroidit à -78°C et on ajoute goutte à goutte, sous bonne agitation, 106 mL d'une solution 1,6M de n-butyllithium (170 mmoles) dans l'hexane dissous dans 100 mL de tétrahydrofurane anhydre. On poursuit l'agitation à -78°C pendant 3 heures puis à -20°C pendant 1 heure. On refroidit à -78°C et on ajoute 130 mL d'une solution d'acide chlorhydrique 1,25M dans l'éthanol. On réchauffe ensuite à température ambiante pendant une heure. On ajoute de l'eau et de l'acétate d'éthyle. On décante la phase organique , on la lave par une solution aqueuse saturée en chlorure de sodium, on la sèche sur sulfate de sodium et on l'évapore à sec. On purifie le résidu par chromatographie sur gel de silice en éluant par du dichlorométhane puis par un mélange 98/2 de dichlorométhane et de méthanol pour obtenir 32,4 g (85,2 mmoles) de produit sous forme d'huile incolore.

### 5.4. 4-[3-(4-chlorophényl)prop-2-yn-1-yl]pipéridine-1-carboxylate de tert-butyle

On dissout 2,29 g (9,6 mmoles) de 1-chloro-4-iodo-benzène et 1,7 mL (12 mmoles) de triéthylamine dans 5 mL de tétrahydrofurane. Sous argon on ajoute 0,076 g (0,40 mmole) d'iodure cuivreux et 0,168 g (0,24 mmole) du complexe de dichlorure de bis(triphénylphoshine) palladium puis, goutte à goutte, une solution de 1,78 g (8 mmoles) de 4-prop-2-yn-1-ylpipéridine-1-carboxylate de tert-butyle, préparé à l'étape 5.3., dans 3 mL de tétrahydrofurane. On poursuit l'agitation pendant une nuit. On ajoute 25 mL d'eau et 100 mL d'acétate d'éthyle. On décante la phase organique, on la lave successivement par 25 mL d'ammoniaque à 10%, 25 mL d'eau et 25 mL d'une solution aqueuse saturée en chlorure de sodium, on la sèche sur sulfate de magnésium et on l'évapore à sec. On purifie le résidu par chromatographie sur gel de silice en éluant par un mélange 95/5 puis 90/10 de cyclohexane et d'acétate d'éthyle pour obtenir 2,15 g (6,44 mmoles) de produit sous forme d'huile jaune.

### 5.5. 4-[3-(4-chlorophényl)prop-2-yn-1-yl]pipéridine

On dissout 2,13 g (6,38 mmoles) de 4-[3-(4-chlorophényl)prop-2-yn-1-yl]pipéridine-1-carboxylate de tert-butyle, obtenu à l'étape 5.4., dans 15 mL de dichlorométhane. On ajoute goutte à goutte une solution de 4,9 mL (63,8 mmoles) d'acide trifluoroacétique dans 5 mL de dichlorométhane. On laisse réagir une nuit à température ambiante puis on évapore à sec. On ajoute 25 mL de dichloroéthane et on réévapore à sec. On reprend ensuite le résidu dans un mélange de 70 mL d'acétate d'éthyle, 10 mL d'une solution aqueuse 1N d'hydroxyde de sodium et 10 mL d'ammoniaque à 30%. On décante la phase organique, on la lave par 2 fois 10 mL d'eau puis par 10 mL d'une solution aqueuse saturée en chlorure de sodium, on la sèche sur sulfate de sodium et on l'évapore à sec pour obtenir 1,39 g (5,94 mmoles) de produit sous forme d'huile brune, utilisé tel quel dans l'étape suivante.

### 5.6. 4-[3-(4-chlorophényl)prop-2-yn-1-yl]pipéridine-1-carboxylate de 2-éthoxy-2-oxoéthyle

On chauffe à 70°C pendant 5 heures, une solution de 1,39 g (5,94 mmoles) de 4-[3-(4-chlorophényl)prop-2-yn-1-yl]pipéridine, préparé à l'étape 5.5. et de 1,86 g (8,33 mmoles) de [(phényloxycarbonyl)oxy]acétate d'éthyle dans 12 mL de toluène. On évapore à sec et on purifie le résidu par chromatographie sur gel de silice en éluant par un mélange 90/10 puis 80/20 de cyclohexane et d'acétate d'éthyle pour obtenir 1,89 g (5,19 mmoles) de produit sous forme d'huile visqueuse.

### 5.7. 4-[3-(4-chlorophényl)prop-2-yn-1-yl]pipéridine-1-carboxylate de 2-(méthylamino)-2-oxoéthyle

On dissout 0,91 g (2,51 mmoles) de 4-[3-(4-chlorophényl)prop-2-yn-1-yl]pipéridine-1-carboxylate de 2-éthoxy-2-oxoéthyle, préparé à l'étape 5.6. dans 4 mL de méthanol. On ajoute 2,5 mL (25 mmoles) d'une solution de méthyl amine à 33% dans l'éthanol et on laisse une nuit à température ambiante. On évapore à sec et on purifie le résidu par chromatographie sur gel de silice en éluant par un mélange 99, 5/0, 5 puis 98/2 et 96/4 de dichlorométhane et de méthanol. On cristallise dans de l'hexane puis on sèche sous vide pour obtenir 0,50 g (1,43 mmoles) de produit sous forme de poudre blanche.
Point de fusion (°C) : 101-103
LC-MS : M+H = 349
RMN-¹H (CDCl₃) δ (ppm) : 7,20 (m, 4H); 6,30 (m, 1H); 4,50 (large s, 2H); 4,10 (large d, 2H); 2,75 (m+d, 5H), 2,30 (d, 2H); 1,85-1,60 (m, 3H); 1,35-1,15 (m, 2H).

### Exemple 6 (composé n°83)

### 4-[3-(4-chlorophényl)propyl]pipérididine-1-carboxylate de 2-(méthylamino)-2-oxoéthyle

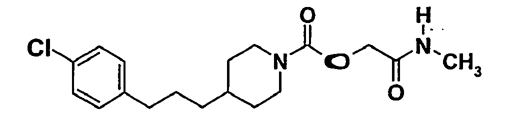

On dissout 0,156 g (0,448 mmole) de 4-[3-(4-chlorophényl)prop-2-yn-1-yl]pipéridine-1-carboxylate de 2-(méthylamino)-2-oxoéthyle, préparé suivant l'exemple 5, dans 2 mL d'éthanol. On ajoute 16 mg de dioxyde de platine. On agite sous atmosphère d'hydrogène à pression et température ambiantes pendant 2 heures puis à 40°C pendant 2 autres heures. On filtre sur célite et on évapore le filtrat. On purifie le résidu par chromatographie HPLC sur gel de Nucleosil en éluant par un gradient 70/30/0 à 0/80/20 d'hexane, d'acétate d'éthyle et de méthanol pour obtenir 0,108 mg (0,306 mmole)de produit sous forme de solide blanc.
Point de fusion (°C) : 118-120
LC-MS : M+H = 353
RMN-¹H (CDCl₃) δ (ppm) : 7,25 (d, 2H) ; 7,10 (d, 2H) ; 6,05 (m, 1H) ; 4,60 (s, 2H); 4,10 (large d, 2H); 2,90 (d, 3H); 2,80 (large t, 2H); 2,60 (t, 2H); 1,75-1,55 (m, 4H); 1,45 (m, 1H) ; 1,35-1,05 (m, 4H).

### Exemple 7 (composé n°74)

### 4-(2-isoquinolin-1-yléthyl)-1-pipéridinecarboxylate de 2-(méthylamino)-2-oxoéthyle

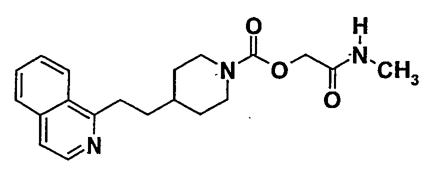

### 7.1. 4-(iodométhyl)-1-pipéridinecarboxylate de tert-butyle

A une solution de 10 g (46,45 mmoles) de 4-(hydroxyméthyl)-1-pipéridinecarboxylate de tert-butyle, de 15,84 g (60,38 mmoles) de triphénylphosphine et de 4,74 g (69,67 mmoles) d'imidazole dans 200 ml de dichlorométhane, refroidie à environ 0°C, on ajoute par petites portions 14,15 g (55,74 mmoles) d'iode (I₂) tout en maintenant la température du milieu réactionnel entre 0°C et 5°C. On poursuit l'agitation à 0°C pendant 1 heure puis à température ambiante pendant 4 heures.

On ajoute 100 ml d'eau et 300 ml d'acétate d'éthyle. On décante la phase organique, on la lave successivement par une solution aqueuse saturée en thiosulfate de sodium et une solution aqueuse saturée en chlorure de sodium, on la sèche sur sulfate de sodium et on concentre sous pression réduite. On purifie le résidu obtenu par chromatographie sur gel de silice en éluant avec un mélange 90/10 de cyclohexane et d'acétate d'éthyle. On obtient 13,70 g (42,13 mmoles) de produit sous forme d'huile incolore.

### 7.2. 4-(2-isoquinolin-1-yléthyl)-1-pipéridinecarboxylate de tert-butyle

A une solution de 2,202 g (15,38 mmoles) de 1-méthylisoquinoline dans 150 ml de tétrahydrofurane, refroidie à environ -70°C, on additionne goutte à goutte 10 ml (20 mmoles) d'une solution (2M) de diisopropylamide de lithium (LDA) dans un mélange de tétrahydrofurane et de n-hexane. On poursuit l'agitation à -70°C pendant 10 minutes puis on additionne lentement une solution de 5 g (15,38 mmoles) de 4-(iodométhyl)-1-pipéridinecarboxylate de tert-butyle, obtenu à l'étape 7.1., dans 30 ml de tétrahydrofurane. Après 30 minutes d'agitation à -70°C, on additionne 100 ml d'une solution aqueuse saturée en chlorure d'ammonium.

On laisse revenir à température ambiante, on sépare la phase aqueuse puis on l'extrait 3 fois avec de l'acétate d'éthyle. On lave les phases organiques réunies avec une solution aqueuse saturée en chlorure de sodium, on les sèche sur sulfate de sodium et on concentre sous pression réduite. On purifie le résidu obtenu par chromatographie sur gel de silice en éluant avec un mélange 99/1 puis 98/2 de dichlorométhane et de méthanol. On obtient 1.80 g (5,29 mmoles) de produit sous forme d'une huile jaune.

### 7.3. (2-pipéridin-4-yléthyl)-1-isoquinoline

A une solution de 1,60 g (4,70 mmoles) de 4-(2-isoquinolin-1-yléthyl)-1-pipéridinecarboxylate de tert-butyle, obtenu à l'étape 7.2., dans 15 ml de 1,4-dioxane, on ajoute à température ambiante, 3,90 ml (23,50 mmoles) d'une solution d'acide chlorhydrique (6N) dans l'isopropanol. On porte ensuite le mélange réactionnel à environ 60°C pendant 12 heures.

On concentre à sec sous pression réduite. On reprend le chlorhydrate obtenu dans 5 ml d'eau puis on additionne lentement, sous agitation, une solution aqueuse à 20% d'hydroxyde de sodium jusqu'à pH 9. On extrait deux fois la phase aqueuse par du chloroforme, on lave les phases organiques réunies avec une solution aqueuse saturée en chlorure de sodium, on les sèche sur sulfate de sodium et on concentre sous pression réduite. On obtient 0,400 g (1,66 mmoles) de produit sous forme d'une huile brune.

### 7.4. 4-(2-isoquinolin-1-yléthyl)-1-pipéridinecarboxylate de 2-éthoxy-2-oxoéthyle

On chauffe à 70°C pendant 18 heures, une solution de 0,320 g (1,33 mmoles) de (2-pipéridin-4-yléthyl)-1-isoquinoline, obtenu à l'étape 7.3., et de 0,388 g (1,73 mmoles) de [(phényloxycarbonyl)oxy]acétate d'éthyle dans 10 ml de toluène.

On laisse revenir à température ambiante, on concentre sous pression réduite puis on purifie le résidu ainsi obtenu par chromatographie sur gel de silice en éluant avec un mélange 40/60 d'acétate d'éthyle et de cyclohexane. On obtient ainsi 0,390 g (1,05 mmoles) de produit sous forme d'une huile visqueuse.

### 7.5. 4-(2-isoquinolin-1-yléthyl)-1-pipéridinecarboxylate de 2-(méthylamino)-2-oxoéthyle

A une solution de 0,380 g (1,03 mmoles) de 4-(2-isoquinolin-1-yléthyl)-1-pipéridinecarboxylate de 2-éthoxy-2-oxoéthyle, préparé à l'étape 7.4., dans 10 ml de méthanol, on ajoute 2,60 ml (5,13 mmoles) d'une solution de méthylamine (2M) dans le tétrahydrofurane. On poursuit l'agitation à température ambiante pendant 12 heures.

Après concentration sous pression réduite, on purifie le résidu obtenu par chromatographie sur gel de silice en éluant avec un mélange 95/5 de dichlorométhane et de méthanol. On obtient un solide que l'on recristallise dans un mélange d'acétate d'éthyle et de diisopropyléther. On obtient ainsi 0,315 g (0,88 mmoles) de produit sous forme de solide blanc.
LC-MS : M+H = 356
Point de fusion (°C) : 126-128
RMN¹H (CDCl₃) δ (ppm) : 8,50 (d, 1H) ; 8,15 (d, 1H) ; 7,90 (d, 1H) ; 7,70 (m, 2H); 7,55 (d, 1H) ; 6,10 (large s, 1H) ; 4,60 (large s, 2H); 4,20 (m, 2H); 3,35 (dd, 2H); 2,90 (m+d, 5H); 1,90 (m, 4H) ; 1,65 (m, 1H) ; 1,30 (m, 2H).

Le tableau qui suit illustre les structures chimiques et les propriétés physiques de quelques composés selon l'invention. Dans ce tableau :
- tous les composés sont sous forme de base libre,
- n-butyle représente un groupe butyle linéaire.

**Tableau**

| | | | | | | | | |
|---|---|---|---|---|---|---|---|---|
| 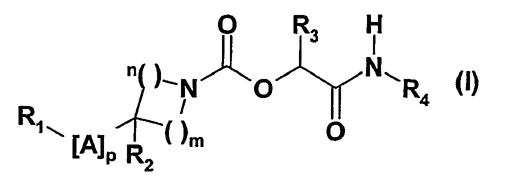 | | | | | | | | |

| n° | R₁ | **[A] ₚ** | **R₂** | **n** | **m** | **R₃** | **R₄** | **PF °C (M+H)** |
|---|---|---|---|---|---|---|---|---|
| 1. | phényl | liaison | H | 2 | 2 | H | H | 160-162 |
| 2. | phényl | liaison | H | 2 | 2 | H | CH₃ | 76-78 |
| 3. | 3-CF₃-phényl | liaison | H | 2 | 2 | H | H | (331) |
| 4. | 3-CF₃-phényl | liaison | H | 2 | 2 | H | CH₃ | (345) |
| 5. | 5-iso-butyl-pyridin-2-yl | liaison | H | 2 | 2 | H | CH₃ | 98-100 |
| 6. | 6-iso-butyl-pyridin-2-yl | liaison | H | 2 | 2 | H | CH₃ | (334) |
| 7. | 6-cyclopentyl-pyridin-2-yl | liaison | H | 2 | 2 | H | CH₃ | (346) |
| 8. | 5-(4-F-phényl)-pyridin-2-yl | liaison | H | 2 | 2 | H | CH₃ | 151-153 |
| 9. | 6-(4-F-phényl)-pyridin-2-yl | liaison | H | 2 | 2 | H | CH₃ | 104-106 |
| 10. | 6-(4-Cl-phényl)-pyridin-2-yl | liaison | H | 2 | 2 | H | CH₃ | 136-138 |
| 11. | 5-(4-CF₃-phényl)-pyridin-2-yl | liaison | H | 2 | 2 | H | CH₃ | 203-205 |
| 12. | 6-(4-CF₃-phényl)-pyridin-2-yl | liaison | H | 2 | 2 | H | CH₃ | 128-130 |
| 13. | 5-(3-CF₃-phényl)-1-méthyl-pyrazol-3-yl | liaison | H | 2 | 2 | H | CH₃ | 160-162 |
| 14. | 4-phényl-imidazol-1-yl | liaison | H | 2 | 2 | H | CH₃ | 192-194 |
| 15. | 5-phényl-1,3,4-oxadiazol-2-yl | liaison | H | 2 | 2 | H | H | 152-154 |
| 16. | 5-phényl-1,3,4-oxadiazol-2-yl | liaison | H | 2 | 2 | H | CH₃ | 114-116 |
| 17. | 5-(4-F-phényl)-1,3,4-oxadiazol-2-yl | liaison | H | 2 | 2 | H | H | 158-160 |
| 18. | 5-(4-F-phényl)-1,3,4-oxadiazol-2-yl | liaison | H | 2 | 2 | H | CH₃ | 163-165 |
| 19. | 5-(3-CF₃-phényl)-1,3,4-oxadiazol-2-yl | liaison | H | 2 | 2 | H | H | 130-130 |
| 20. | 5- (3-CF₃-phényl)-1,3,4-oxadiazol-2-yl | liaison | H | 2 | 2 | H | CH₃ | 123-125 |
| 21. | 3- (3-CF₃-phényl)-1,2,4-oxadiazol-5-yl | liaison | H | 2 | 2 | H | H | 133 -135 |
| 22. | 3- (3-CF₃-phényl)-1,2,4-oxadiazol-5-yl | liaison | H | 2 | 2 | H | CH₃ | 119 -121 |
| 23. | benzoxazol-2-yl | liaison | H | 2 | 2 | H | CH₃ | 137 -139 |
| 24. | benzothiazol-2-yl | liaison | H | 2 | 2 | H | H | 148 -150 |
| 25. | benzothiazol-2-yl | liaison | H | 2 | 2 | H | CH₃ | 120 -122 |
| 26. | benzimidazol-2-yl | liaison | H | 2 | 2 | H | CH₃ | 213-215 |
| 27. | benzimidazol-1-yl | liaison | H | 2 | 2 | H | H | 206-208 |
| 28. | 2-phényl-benzimidazol-1-yl | liaison | H | 2 | 2 | H | CH₃ | 193 -195 |
| 29. | benzotriazol-1-yl | liaison | H | 2 | 2 | H | CH₃ | 129-131 |
| 30. | 5-CF₃-benzotriazol-1-yl | liaison | H | 2 | 2 | H | H | 152-154 |
| 31. | indol-1-yl | liaison | H | 2 | 2 | H | H | 178-180 |
| 32. | 4-Br-phényl | liaison | OH | 2 | 2 | H | CH₃ | 57 -60 |
| 33. | 4-(4-F-phényl)phényl | liaison | OH | 2 | 2 | H | CH₃ | 212-214 |
| 34. | 4-(4-Cl-phényl)phényl | liaison | OH | 2 | 2 | H | CH₃ | 223-225 |
| 35. | 4-(4-CH₃-phényl)phényl | liaison | OH | 2 | 2 | H | CH₃ | 179-181 |
| 36. | 4-(4-n-butyl-phényl)phényl | liaison | OH | 2 | 2 | H | CH₃ | (425) |
| 37. | 4-(4-CF₃-phényl)phényl | liaison | OH | 2 | 2 | H | CH₃ | 191-193 |
| 38. | 4-(4-CH₃O phényl)phényl | liaison | OH | 2 | 2 | H | CH₃ | 175-176 |
| | 39. 4-(4-C₂H₅O-phényl)phényl | liaison | OH | 2 | 2 | H | CH₃ | 165-167 |
| 40. | 4-(3-Cl,4-Cl-phényl)phényl | liaison | OH | 2 | 2 | H | CH₃ | 156-158 |
| 41. | 4-(3-F,4-CH₃O-phényl)phényl | liaison | OH | 2 | 2 | H | CH₃ | (417) |
| 42. | 4-(3-Cl,4-F-phényl)phényl | liaison | OH | 2 | 2 | H | CH₃ | 123- 125 |
| 43: | naphthalèn-2-yl | CH₂ | H | 2 | 2 | H | CH₃ | 150-152 |
| 44. | 4-phényl-phényl | CH₂ | H | 2 | 2 | H | CH₃ | 115-117 |
| 45. | 6-cyclopentyl-pyridin-2-yl | CH₂ | H | 2 | 2 | H | CH₃ | (360) |
| 46. | 6-(4-F-phényl)-pyridin-2-yl | CH₂ | H | 2 | 2 | H | CH₃ | 112-114 |
| 47. | indol-1-yl | CH₂ | H | 2 | 2 | H | H | 158-159 |
| 48. | indolin-1-yl | CH₂ | H | 2 | 2 | H | H | 115-116 |
| 49. | 1,2,3,4-tétrahydroquinolin-1-yl | CH₂ | H | 2 | 2 | H | H | 158-159 |
| 50. | 1,2,3,4-tétrahydroisoquinolin -2-yl | CH₂ | H | 2 | 2 | H | H | (332) |
| 51. | pyrrolo[2,3-b] pyridin-1-yl | CH₂ | H | 2 | 2 | H | H | (317) |
| 52. | benzimidazol-1-yl | CH₂ | H | 2 | 2 | H | H | (317) |
| 53. | 4-phénylimidazol-1-yl | CH₂ | H | 2 | 2 | H | H | 124- 125 |
| 54. | phényl | (CH₂)₂ | H | 1 | 2 | H | CH₃ | (291) |
| 55. | 4-F-phényl | (CH₂)₂ | H | 2 | 2 | H | CH₃ | 150-152 |
| 56. | 3-Cl-phényl | (CH₂)₂ | H | 2 | 2 | H | CH₃ | 86-88 |
| 57. | 4-Cl-phényl | (CH₂)₂ | H | 2 | 2 | H | CH₃ | 150-152 |
| | 58. 3-CF₃-phényl | (CH₂)₂ | H | 2 | 2 | H | CH₃ | 103-105 |
| 59. | 4-CF₃-phényl | (CH₂)₂ | H | 2 | 2 | H | CH₃ | 131-133 |
| 60. | 3-CN-phényl | (CH₂)₂ | H | 2 | 2 | H | CH₃ | (330) |
| 61. | 4-CH₃-phényl | (CH₂)₂ | H | 2 | 2 | H | H | 125-127 |
| 62. | 4-CH₃-phényl | (CH₂)₂ | H | 2 | 2 | H | CH₃ | 117-119 |
| 63. | 4-CH₃O-phényl | (CH₂)₂ | H | 2 | 2 | H | H | 123-125 |
| 64. | 4-CH₃O-phényl | (CH₂)₂ | H | 2 | 2 | H | CH₃ | 122-124 |
| 65. | 2-phényl-phényl | (CH₂)₂ | H | 2 | 2 | H | CH₃ | (381) |
| 66. | 3-phényl-phényl | (CH₂)₂ | H | 2 | 2 | H | CH₃ | 113-115 |
| 67. | naphtalèn-1-yl | (CH₂)₂ | H | 2 | 2 | H | CH₃ | 112-114 |
| 68. | naphtalèn-2-yl | (CH₂)₂ | H | 2 | 2 | H | CH₃ | 106-108 |
| 69. | pyrimidin-2-yl | (CH₂)₂ | H | 2 | 2 | H | CH₃ | 160-170 |
| 70. | pyrimidin-5-yl | (CH₂)₂ | H | 2 | 2 | H | CH₃ | 123-125 |
| 71. | 6-cyclopentyl-pyridin-2-yl | (CH₂)₂ | H | 2 | 2 | H | CH₃ | (374) |
| 72. | 6-pyrrolidin-1-ylpyridin-2-yl | (CH₂)₂ | H | 2 | 2 | H | CH₃ | 130-132 |
| 73. | thiazol-2-yl | (CH₂)₂ | H | 2 | 2 | H | CH₃ | 97-99 |
| 74. | isoquinolin-1-yl | (CH₂)₂ | H | 2 | 2 | H | CH₃ | 126-128 |
| 75. | 1,2,3,4-tétrahydroquinolin-1-yl | (CH₂)₂ | H | 2 | 2 | H | H | (346) |
| 76. | 1,2,3,4-tétrahydroisoquinolin-2-yl | (CH₂)₂ | H | 2 | 2 | H | H | 112-114 |
| 77. | indol-1-yl | (CH₂)₂ | H | 2 | 2 | H | H | (330) |
| 78. | indolin-1-yl | (CH₂)₂ | H | 2 | 2 | H | H | 92-93 |
| 79. | pyrrolo[2,3-b]pyridin-1-yl | (CH₂)₂ | H | 2 | 2 | H | H | (331) |
| 80. | benzimidazol-1-yl | (CH₂)₂ | H | 2 | 2 | H | H | 181-182 |
| 81. | 4-phénylimidazol-1-yl | (CH₂)₂ | H | 2 | 2 | H | H | 183-184 |
| 82. | 3-Cl-phényl | (CH₂)₃ | H | 2 | 2 | H | CH₃ | 92-94 |
| 83. | 4-Cl-phényl | (CH₂)₃ | H | 2 | 2 | H | CH₃ | 118-120 |
| 84. | 3-CF₃-phényl | (CH₂)₃ | H | 2 | 2 | H | CH₃ | 106-108 |
| 85. | 4-CF₃-phényl | (CH₂)₃ | H | 2 | 2 | H | CH₃ | 111-113 |
| 86. | 3-CN-phényl | (CH₂)₃ | H | 2 | 2 | H | CH₃ | 118-120 |
| 87. | 2-phényl-phényl | (CH₂)₃ | H | 2 | 2 | H | CH₃ | (395) |
| 88. | 3-phényl-phényl | (CH₂)₃ | H | 2 | 2 | H | CH₃ | 116-118 |
| 89. | napthalèn-1-yl | (CH₂)₃ | H | 2 | 2 | H | CH₃ | (369) |
| 90. | napthalèn-2-yl | (CH₂)₃ | H | 2 | 2 | H | CH₃ | 112-114 |
| 91. | pyrimidin-2-yl | (CH₂)₃ | H | 2 | 2 | H | CH₃ | 105-107 |
| 92. | pyrimidin-5-yl | (CH₂)₃ | H | 2 | 2 | H | CH₃ | 105-107 |
| 93. | thiazol-2-yl | (CH₂)₃ | H | 2 | 2 | H | CH₃ | (326) |
| 94. | 3-Cl-phényl | C≡C | H | 2 | 2 | H | CH₃ | 85-87 |
| 95. | 4-C1-phényl | C≡C | H | 2 | 2 | H | CH₃ | 122-124 |
| 96. | 3-CF₃-phényl | C≡C | H | 2 | 2 | H | CH₃ | (369) |
| 97. | 4-CF₃-phényl | C≡C | H | 2 | 2 | H | CH₃ | 134-136 |
| 98. | 3-CN-phényl | C≡C | H | 2 | 2 | H | CH₃ | (326) |
| 99. | 2-phényl-phényl | C≡C | H | 2 | 2 | H | CH₃ | (377) |
| 100 | 3-phényl-phényl | C≡C | H | 2 | 2 | H | CH₃ | (377) |
| 101 | naphtalèn-1-yl | C≡C | H | 2 | 2 | H | CH₃ | (351) |
| 102 | naphtalèn-2-yl | C≡C | H | 2 | 2 | H | CH₃ | (351) |
| 103 | pyrimidin-2-yl | C≡C | H | 2 | 2 | H | CH₃ | (303) |
| 104 | pyrimidin-5-yl | C≡C | H | 2 | 2 | H | CH₃ | 136-138 |
| 105 | thiazol-2-yl | C≡C | H | 2 | 2 | H | CH₃ | (308) |
| 106 | 3-Cl-phényl | C≡CCH₂ | H | 2 | 2 | H | CH₃ | 91-93 |
| 107 | 4-Cl-phényl | C≡CCH₂ | H | 2 | 2 | H | CH₃ | 101-103 |
| 108 | 3-CF₃-phényl | C≡CCH₂ | H | 2 | 2 | H | CH₃ | 113-115 |
| 109 | 4-CF₃-phényl | C≡CCH₂ | H | 2 | 2 | H | CH₃ | 112-114 |
| 110 | 3-CN-phényl | C≡CCH₂ | H | 2 | 2 | H | CH₃ | 112-114 |
| 111 | 2-phényl-phényl | C≡CCH₂ | H | 2 | 2 | H | CH₃ | 99-101 |
| 112 | 3-phényl-phényl | C≡CCH₂ | H | 2 | 2 | H | CH₃ | (391) |
| 113 | naphtalèn-1-yl | C≡CCH₂ | H | 2 | 2 | H | CH₃ | 98-100 |
| 114 | naphtalèn-2-yl | C≡CCH₂ | H | 2 | 2 | H | CH₃ | 99-101 |
| 115 | pyrimidin-2-yl | C≡CCH₂ | H | 2 | 2 | H | CH₃ | 91-93 |
| 116 | pyrimidin-5-yl | C≡CCH₂ | H | 2 | 2 | H | CH₃ | 113-115 |
| 117 | thiazol-2-yl | C≡CCH₂ | H | 2 | 2 | H | CH₃ | 112-114 |
| 118 | 6-pyrrolidin-1-ylpyridin-2-yl | CH₂ | H | 2 | 2 | H | CH₃ | 119-121 |
| 119 | 6-(1-isopropylpiperidin-4-yl)pyridin-2-yl | (CH₂)₂ | H | 2 | 2 | H | CH₃ | (431) |

Les composés de l'invention ont fait l'objet d'essais pharmacologiques permettant de déterminer leur effet inhibiteur de l'enzyme FAAH (Fatty Acid amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005 et Journal of Pharmacology and Experimental Therapeutics (1997), 283, 729-734). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est comptée par scintillation liquide.

Dans ces conditions, les composés les plus actifs de l'invention présentent des CI₅₀ (concentration inhibant de 50% l'activité enzymatique contrôle de la FAAH) comprises entre 0,001 et 1 µM.

Par exemple, les composés n°39 et 40 du tableau présentent des CI₅₀ de respectivement 0,095 et 0,098 µM.

Il apparaît donc que les composés selon l'invention ont une activité inhibitrice sur l'enzyme FAAH.

L'activité *in vivo* des composés de l'invention a été évaluée dans un test d'analgésie.

Ainsi, l'administration intrapéritonéale (i.p.) de PBQ (phénylbenzoquinone, 2 mg/kg dans une solution de chlorure de sodium à 0,9% contenant 5% d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5%, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg. Par exemple, le composé n° 57 du tableau réduit de 37% et de 74% le nombre d'étirements induits par le PBQ, à la dose de 3 mg/kg p.o., respectivement à 60 minutes et à 120 minutes.

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyléthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.

Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoides endogènes et/ ou tous autres substrats métabolisés par l'enzyme FAAH, sont impliqués.

On peut par exemple citer les maladies et les affections suivantes :
La douleur notamment les douleurs aiguës ou chroniques de type neurogène : migraine, douleurs neuropathiques incluant les formes associées au virus de l'herpès et au diabète ;
les douleurs aiguës ou chroniques associées aux maladies inflammatoires : arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie ;
les troubles du comportement alimentaire en particulier les anorexies et cachexies de diverses natures ;
les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses ;
les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires ;
l'épilepsie ;
les troubles du sommeil incluant les apnées du sommeil ;
les maladies cardiovasculaires en particulier hypertension, arythmies cardiaques, artériosclérose, crise cardiaque, ischémies cardiaques ;
l'ischémie rénale ;
les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
les désordres du système immunitaire, notamment les maladies auto-immunes : psoriasis, lupus érythémateux, maladies du tissu conjonctif ou connectivites, syndrome de Sjögren's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire ;
les maladies allergiques : l'hypersensibilité immédiate ou retardée, rhinites ou conjonctivites allergiques, dermatites de contact ;
les maladies infectieuses parasitaires , virales ou bactériennes : SIDA, méningites ; les maladies inflammatoires, notamment les maladies articulaires : arthrite, arthrite rhumatoïde, ostéoarthrite, spondylite, goutte, vascularite, maladie de Crohn, syndrome du colon irritable ; l'ostéoporose ; les affections oculaires : hypertension oculaire, glaucome ;
les affections pulmonaires : maladies des voies respiratoires, bronchospasmes, toux, asthme, bronchite chronique, obstruction chronique des voies respiratoires, emphysème ;
les maladies gastro-intestinales: syndrome du colon irritable, désordres inflammatoires intestinaux, ulcères, diarrhées ;
l'incontinence urinaire et l'inflammation vésicale.

L'utilisation d'un composé de formule (I), à l'état de base, de sel, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel, ou un hydrate, ou un solvat pharmaceutiquement acceptable dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de mais | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

## Revendications

1. Composé répondant à la formule générale (I) : 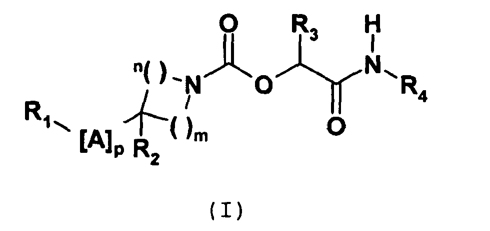 choisi parmi les composés du tableau suivant :
| n° | R₁ | **[A]ₚ** | **R₂** | **n** | **m** | **R₃** | **R₄** |
|---|---|---|---|---|---|---|---|
| 1. | phényl | liaison | H | 2 | 2 | H | H |
| 2. | phényl | liaison | H | 2 | 2 | H | CH₃ |
| 3. | 3-CF₃-phényl | liaison | H | 2 | 2 | H | H |
| 4. | 3-CF₃-phényl | liaison | H | 2 | 2 | H | CH₃ |
| 5. | 5-iso-butyl-pyridin-2-yl | liaison | H | 2 | 2 | H | CH₃ |
| 6. | 6-iso-butyl-pyridin-2-yl | liaison | H | 2 | 2 | H | CH₃ |
| 7. | 6-cyclopentyl-pyridin-2-yl | liaison | H | 2 | 2 | H | CH₃ |
| 8. | 5-(4-F-phényl)-pyridin-2-yl | liaison | H | 2 | 2 | H | CH₃ |
| 9. | 6-(4-F-phényl)-pyridin-2-yl | liaison | H | 2 | 2 | H | CH₃ |
| 10. | 6-(4-Cl-phényl)-pyridin-2-yl | liaison | H | 2 | 2 | H | CH₃ |
| 11. | 5-(4-CF₃-phényl)-pyridin-2-yl | liaison | H | 2 | 2 | H | CH₃ |
| 12. | 6-(4-CF₃-phényl)-pyridin-2-yl | liaison | H | 2 | 2 | H | CH₃ |
| 13. | 5-(3-CF₃-phényl)-1-méthyl-pyrazol-3-yl | liaison | H | 2 | 2 | H | CH₃ |
| 14. | 4-phényl-imidazol-1-yl | liaison | H | 2 | 2 | H | CH₃ |
| 15. | 5-phényl-1,3,4-oxadiazol-2-yl | liaison | H | 2 | 2 | H | H |
| 16. | 5-phényl-1,3,4-oxadiazol-2-yl | liaison | H | 2 | 2 | H | CH₃ |
| 17. | 5-(4-F-phényl)-1,3,4-oxadiazol-2-yl | liaison | H | 2 | 2 | H | H |
| 18. | 5-(4-F-phényl)-1,3,4-oxadiazol-2-yl | liaison | H | 2 | 2 | H | CH₃ |
| 19. | 5-(3-CF₃-phényl)-1,3,4-oxadiazol-2-yl | liaison | H | 2 | 2 | H | H |
| 20. | 5-(3-CF₃-phényl)-1,3,4-oxadiazol-2-yl | liaison | H | 2 | 2 | H | CH₃ |
| 21. | 3-(3-CF₃-phényl)-1,2,9-oxadiazol-5-yl | liaison | H | 2 | 2 | H | H |
| 22. | 3-(3-CF₃-_{P}hényl)-1,2,4-oxadiazol-5-yl | liaison | H | 2 | 2 | H | CH₃ |
| 23. | benzoxazol-2-yl | liaison | H | 2 | 2 | H | CH₃ |
| 24. | benzothiazol-2-yl | liaison | H | 2 | 2 | H | H |
| 25. | benzothiazol-2-yl | liaison | H | 2 | 2 | H | CH₃ |
| 26. | benzimidazol-2-yl | liaison | H | 2 | 2 | H | CH₃ |
| 27. | benzimidazol-1-yl | liaison | H | 2 | 2 | H | H |
| 28. | 2-phényl-benzimidazol-1-yl | liaison | H | 2 | 2 | H | CH₃ |
| 29. | benzotriazol-1-yl | liaison | H | 2 | 2 | H | CH₃ |
| 30. | 5-CF₃-benzotriazol-1-yl | liaison | H | 2 | 2 | H | H |
| 31. | indol-1-yl | liaison | H | 2 | 2 | H | H |
| 32. | 4-Br-phényl | liaison | OH | 2 | 2 | H | CH₃ |
| 33. | 4-(4-F-phényl)phényl | liaison | OH | 2 | 2 | H | CH₃ |
| 34. | 4-(4-Cl-phényl)phényl | liaison | OH | 2 | 2 | H | CH₃ |
| 35. | 4-(4-CH₃-phényl)phényl | liaison | OH | 2 | 2 | H | CH₃ |
| 36. | 4-(4-n-butyl-phényl)phényl | liaison | OH | 2 | 2 | H | CH₃ |
| 37. | 4- (4-CF₃-phényl) phényl | liaison | OH | 2 | 2 | H | CH₃ |
| 38. | 4-(4-CH₃O-phényl)phényl | liaison | OH | 2 | 2 | H | CH₃ |
| 39. | 4-(4-C₂H₅O-phényl)phényl | liaison | OH | 2 | 2 | H | CH₃ |
| 40. | 4-(3-Cl,4-Cl-phényl)phényl | liaison | OH | 2 | 2 | H | CH₃ |
| 41. | 4-(3-F,4-CH₃O-phényl)phényl | liaison | OH | 2 | 2 | H | CH₃ |
| 42. | 4-(3-C1,4-F-phényl)phényl | liaison | OH | 2 | 2 | H | CH₃ |
| 43. | naphthalèn-2-yl | CH₂ | H | 2 | 2 | H | CH₃ |
| 44. | 4-phényl-phényl | CH₂ | H | 2 | 2 | H | CH₃ |
| 45. | 6-cyclopentyl-pyridin-2-yl | CH₂ | H | 2 | 2 | H | CH₃ |
| 46. | 6-(4-F-phényl)-pyridin-2-yl | CH₂ | H | 2 | 2 | H | CH₃ |
| 47. | indol-1-yl | CH₂ | H | 2 | 2 | H | H |
| 48. | indolin-1-yl | CH₂ | H | 2 | 2 | H | H |
| 49. | 1,2,3,4-tétrahydroquinolin-1-yl | CH₂ | H | 2 | 2 | H | H |
| 50. | 1,2,3,4-tétrahydroisoquinolin-2-yl | CH₂ | H | 2 | 2 | H | H |
| 51. | pyrrolo[2,3-b]pyridin-1-yl | CH₂ | H | 2 | 2 | H | H |
| 52. | benzimidazol-1-yl | CH₂ | H | 2 | 2 | H | H |
| 53. | 4-phénylimidazol-1-yl | CH₂ | H | 2 | 2 | H | H |
| 54. | phényl | (CH₂)₂ | H | 1 | 2 | H | CH₃ |
| 55. | 4-F-phényl | (CH₂)₂ | H | 2 | 2 | H | CH₃ |
| 56. | 3-Cl-phényl | (CH₂)₂ | H | 2 | 2 | H | CH₃ |
| 57. | 4-Cl-phényl | (CH₂)₂ | H | 2 | 2 | H | CH₃ |
| 58. | 3-CF₃-phényl | (CH₂)₂ | H | 2 | 2 | H | CH₃ |
| 59. | 4-CF₃-phényl | (CH₂)₂ | H | 2 | 2 | H | CH₃ |
| 60. | 3-CN-phényl | (CH₂)₂ | H | 2 | 2 | H | CH₃ |
| 61. | 4-CH₃-phényl | (CH₂)₂ | H | 2 | 2 | H | H |
| 62. | 4-CH₃-phényl | (CH₂)₂ | H | 2 | 2 | H | CH₃ |
| 63. | 4-CH₃O-phényl | (CH₂)₂ | H | 2 | 2 | H | H |
| 64. | 4-CH₃O-phényl | (CH₂)₂ | H | 2 | 2 | H | CH₃ |
| 65. | 2-phényl-phényl | (CH₂)₂ | H | 2 | 2 | H | CH₃ |
| 66. | 3-phényl-phényl | (CH₂)₂ | H | 2 | 2 | H | CH₃ |
| 67. | naphtalèn-1-yl | (CH₂)₂ | H | 2 | 2 | H | CH₃ |
| 68. | naphtalèn-2-yl | (CH₂)₂ | H | 2 | 2 | H | CH₃ |
| 69. | pyrimidin-2-yl | (CH₂)₂ | H | 2 | 2 | H | CH₃ |
| 70. | pyrimidin-5-yl | (CH₂)₂ | H | 2 | 2 | H | CH₃ |
| 71. | 6-cyclopentyl-pyridin-2-yl | (CH₂)₂ | H | 2 | 2 | H | CH₃ |
| 72. | 6-pyrrolidin-1-ylpyridin-2-yl | (CH₂)₂ | H | 2 | 2 | H | CH₃ |
| 73. | thiazol-2-yl | (CH₂)₂ | H | 2 | 2 | H | CH₃ |
| 74. | isoquinolin-1-yl | (CH₂)₂ | H | 2 | 2 | H | CH₃ |
| 75. | 1,2,3,4-tétrahydroquinolin-1-yl | (CH₂)₂ | H | 2 | 2 | H | H |
| 76. | 1,2,3,4-tétrahydroisoquinolin-2-yl | (CH₂)₂ | H | 2 | 2 | H | H |
| 77. | indol-1-yl | (CH₂)₂ | H | 2 | 2 | H | H |
| 78. | indolin-1-yl | (CH₂)₂ | H | 2 | 2 | H | H |
| 79. | pyrrolo[2,3-b]pyridin-1-yl | (CH₂)₂ | H | 2 | 2 | H | H |
| 80. | benzimidazol-1-yl | (CH₂)₂ | H | 2 | 2 | H | H |
| 81. | 4-phénylimidazol-1-yl | (CH₂)₂ | H | 2 | 2 | H | H |
| 82. | 3-Cl-phényl | (CH₂)₃ | H | 2 | 2 | H | CH₃ |
| 83. | 4-Cl-phényl | (CH₂)₃ | H | 2 | 2 | H | CH₃ |
| 84. | 3-CF₃-phényl | (CH₂)₃ | H | 2 | 2 | H | CH₃ |
| 85. | 4-CF₃-phényl | (CH₂)₃ | H | 2 | 2 | H | CH₃ |
| 86. | 3-CN-phényl | (CH₂)₃ | H | 2 | 2 | H | CH₃ |
| 87. | 2-phényl-phényl | (CH₂)₃ | H | 2 | 2 | H | CH₃ |
| 88. | 3-phényl-phényl | (CH₂)₃ | H | 2 | 2 | H | CH₃ |
| 89. | napthalèn-1-yl | (CH₂)₃ | H | 2 | 2 | H | CH₃ |
| 90. | napthalèn-2-yl | (CH₂)₃ | H | 2 | 2 | H | CH₃ |
| 91. | pyrimidin-2-yl | (CH₂)₃ | H | 2 | 2 | H | CH₃ |
| 92. | pyrimidin-5-yl | (CH₂)₃ | H | 2 | 2 | H | CH₃ |
| 93. | thiazol-2-yl | (CH₂)₃ | H | 2 | 2 | H | CH₃ |
| 94. | 3-Cl-phényl | C≡C | H | 2 | 2 | H | CH₃ |
| 95. | 4-Cl-phényl | C≡C | H | 2 | 2 | H | CH₃ |
| 96. | 3-CF₃-phényl | C≡C | H | 2 | 2 | H | CH₃ |
| 97. | 4-CF₃-phényl | C≡C | H | 2 | 2 | H | CH₃ |
| 98. | 3-CN-phényl | C≡C | H | 2 | 2 | H | CH₃ |
| 99. | 2-phényl-phényl | C≡C | H | 2 | 2 | H | CH₃ |
| 100 | 3-phényl-phényl | C≡C | H | 2 | 2 | H | CH₃ |
| 101 | naphtalèn-1-yl | C≡C | H | 2 | 2 | H | CH₃ |
| 102 | naphtalèn-2-yl | C≡C | H | 2 | 2 | H | CH₃ |
| 103 | pyrimidin-2-yl | C≡C | H | 2 | 2 | H | CH₃ |
| 104 | pyrimidin-5-yl | C≡C | H | 2 | 2 | H | CH₃ |
| 105 | thiazol-2-yl | C≡C | H | 2 | 2 | H | CH₃ |
| 106 | 3-Cl-phényl | C≡CCH₂ | H | 2 | 2 | H | CH₃ |
| 107 | 4-Cl-phényl | C≡CCH₂ | H | 2 | 2 | H | CH₃ |
| 108 | 3-CF₃-phényl | C≡CCH₂ | H | 2 | 2 | H | CH₃ |
| 109 | 4-CF₃-phényl | C≡CCH₂ | H | 2 | 2 | H | CH₃ |
| 110 | 3-CN-phényl | C≡CCH₂ | H | 2 | 2 | H | CH₃ |
| 111 | 2-phényl-phényl | C≡CCH₂ | H | 2 | 2 | H | CH₃ |
| 112 | 3-phényl-phényl | C≡CCH₂ | H | 2 | 2 | H | CH₃ |
| 113 | naphtalèn-1-yl | C≡CCH₂ | H | 2 | 2 | H | CH₃ |
| 114 | naphtalèn-2-yl | C≡CCH₂ | H | 2 | 2 | H | CH₃ |
| 115 | pyrimidin-2-yl | C≡CCH₂ | H | 2 | 2 | H | CH₃ |
| 116 | pyrimidin-5-yl | C≡CCH₂ | H | 2 | 2 | H | CH₃ |
| 117 | thiazol-2-yl | C≡CCH₂ | H | 2 | 2 | H | CH₃ |
| 118 | 6-pyrrolidin-1-ylpyridin-2-yl | CH₂ | H | 2 | 2 | H | CH₃ |
| 119 | 6-(1-isopropylpiperidin-4-yl)pyridin-2-yl | (CH₂)₂ | H | 2 | 2 | H | CH₃ |
**à l'état de base, de sel d'addition à un acide, d'hydrate ou de solvat.**

2. Procédé de préparation d'un composé de formule (I) selon la revendication 1, comprenant l'étape consistant à transformer le carbamate-ester de formule générale (IV) 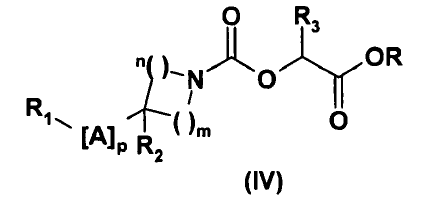 dans laquelle dans laquelle R₁, A, R₂, R₃, p, m et n sont tels que définis dans la formule (I) selon la revendication 1 et R représente un groupe méthyle ou éthyle,
par aminolyse au moyen d'une amine de formule générale R₄NH₂ dans laquelle R₄ est tel que défini dans la formule (I) selon la revendication 1.

3. Composé répondant à la formule générale (IV), 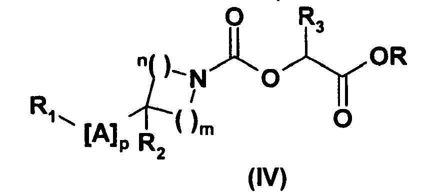 dans laquelle R₁, A, R₂, R₃, p, m et n sont tels que définis dans la formule (I) selon la revendication 1 et R représente un groupe méthyle ou éthyle.

4. Médicament comprenant un composé de formule (I) selon la revendication 1, ou un sel, ou encore un hydrate ou solvat pharmaceutiquement acceptable du composé de formule (I).

5. Composition pharmaceutique contenant au moins un composé de formule (I) selon la revendication 1, à l'état de base, de sel, d'hydrate ou de solvat pharmaceutiquement acceptable et éventuellement un ou plusieurs excipients pharmaceutiquement acceptables.

6. Composé de formule (I) selon la revendication 1, à l'état de base, de sel, d'hydrate ou de solvat pharmaceutiquement acceptable, pour son utilisation comme médicament.

7. Utilisation d'un composé de formule (I) selon la revendication 1, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale.

## Claims

1. Compound corresponding to the general formula (I) 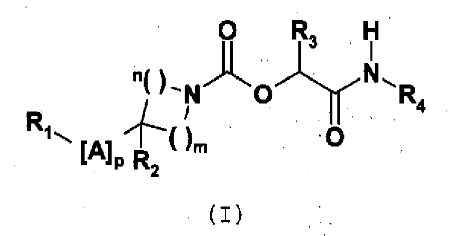 chosen from the compounds of the following table:
| **No.** | **R₁** | **[A] ₚ** | **R₂** | **n** | **m** | **R₃** | **R₄** |
|---|---|---|---|---|---|---|---|
| 1. | phenyl | bond | H | 2 | 2 | H | H |
| 2. | phenyl | bond | H | 2 | 2 | H | CH₃ |
| 3. | 3-CF₃-phenyl | bond | H | 2 | 2 | H | H |
| 4. | 3-GF₃-phenyl | bond | H | 2 | 2 | H | CH₃ |
| 5. | 5-isobutylpyrid-2-yl | bond | H | 2 | 2 | H | CH₃ |
| 6. | 6-isobutylpyrid-2-yl | bond | H | 2 | 2 | H | CH₃ |
| 7. | 6-cyclopentylpyrid-2-yl | bond | H | 2 | 2 | H | CH₃ |
| 8. | 5-(4-F-phenyl)pyrid-2-yl | bond | H | 2 | 2 | H | CH₃ |
| 9. | 6-(4-F-phenyl)pyrid-2-yl | bond | H | 2 | 2 | H | CH₃ |
| 10. | 6-(4-Cl-phenyl)pyrid-2-yl | bond | H | 2 | 2 | H | CH₃ |
| 11. | 5-(4-CF3-phenyl)pyrid-2-yl | bond | H | 2 | 2 | H | CH₃ |
| 12. | 6- (4-CF₃-phenyl)pyrid-2-yl | bond | H | 2 | 2 | H | CH₃ |
| 13. | 5-(3-CF₃-phenyl)-1-methylpyrazol-3-yl | bond | H | 2 | 2 | H | CH₃ |
| 14. | 4-phenylimidazol-1-yl | bond | H | 2 | 2 | H | CH₃ |
| 15. | 5-phenyl-1,3,4-oxadiazol-2-yl | bond | H | 2 | 2 | H | H |
| 16. | 5-phenyl-1,3,4-oxadiazol-2-yl | bond | H | 2 | 2 | H | CH₃ |
| 17. | 5-(4-F-phenyl)-1,3,4-oxa-diazol-2-yl | bond | H | 2 | 2 | H | H |
| 18. | 5-(4-F-phenyl)-1,3,4-oxa-diazol-2-yl | bond | H | 2 | 2 | H | CH₃ |
| 19. | 5-(3-CF₃-phenyl)-1,3,4-oxadiazol-2-yl | bond | H | 2 | 2 | H | H |
| 20. | 5- (3-CF₃-phenyl)-1,3,4-oxadiazol-2-yl | bond | H | 2 | 2 | H | CH₃ |
| 21. | 3-(3-CF3-phenyl)-1,2,4-oxadiazol-5-yl | bond | H | 2 | 2 | H | H |
| 22. | 3- (3-CF₃-phenyl)-1,2,4-oxadiazol-5-yl | bond | H | 2 | 2 | H | CH₃ |
| 23. | benzoxazol-2-yl | bond | H | 2 | 2 | H | CH₃ |
| 24. | benzothiazol-2-yl | bond | H | 2 | 2 | H | H |
| 25. | benzothiazol-2-yl | bond | H | 2 | 2 | H | CH₃ |
| 26. | benzimidazol-2-yl | bond | H | 2 | 2 | H | CH₃ |
| 27. | benzimidazol-1-yl | bond | H | 2 | 2 | H | H |
| 28. | 2-phenylvbenzimidazol-1-yl | bond | H | 2 | 2 | H | CH₃ |
| 29. | benzotriazol-1-yl | bond | H | 2 | 2 | H | CH₃ |
| 30. | 5-CF₃-benzotriazol-1-yl | bond | H | 2 | 2 | H | H |
| 31. | indol-1-yl | bond | H | 2 | 2 | H | H |
| 32. | 4-Br-phenyl | bond | OH | 2 | 2 | H | CH₃ |
| 33. | 4-(4-F-phenyl)phenyl | bond | OH | 2 | 2 | H | CH₃ |
| 34. | 4-(4-Cl-phenyl)phenyl | bond | OH | 2 | 2 | H | CH₃ |
| 35. 4- | (4-CH₃-phenyl) phenyl | bond | OH | 2 | 2 | H | CH₃ |
| 36. | 4-(4-(n-butyl)phenyl)-phenyl | bond | OH | 2 | 2 | H | CH₃ |
| 37. | 4-(4-CF₃-phenyl)phenyl | bond | CH | 2 | 2 | H | CH₃ |
| 38. | 4-(4-CH₃O-phenyl)phenyl | bond | OH | 2 | 2 | H | CH₃ |
| 39. | 4-(4-C₂H₅O-phenyl)phenyl | bond | OH | 2 | 2 | H | CH₃ |
| 40. | 4-(3-Cl, 4-Cl-phenyl)-phenyl | bond | OH | 2 | 2 | H | CH₃ |
| 41. | 4-(3-F, 4-CH₃O-phenyl)-phenyl | bond | OH | 2 | 2 | H | CH₃ |
| 42. | 4-(3-Cl, 4-F-phenyl)phenyl | bond | OH | 2 | 2 | H | CH₃ |
| 43. | naphth-2-yl | CH₂ | H | 2 | 2 | H | CH₃ |
| 44. | 4-phenylphenyl | CH₂ | H | 2 | 2 | H | CH₃ |
| 45. | 6-cyclopentylpyrid-2-yl | CH₂ | H | 2 | 2 | H | CH₃ |
| 46. | 6-(4-F-phenyl)pyrid-2-yl | CH₂ | H | 2 | 2 | H | CH₃ |
| 47. | indol-l-yl | CH₂ | H | 2 | 2 | H | H |
| 48. | indolin-l-yl | CH₂ | H | 2 | 2 | H | H |
| 49. | 1,2,3,4-tetrahydro-quinolin-1-yl | CH₂ | H | 2 | 2 | H | H |
| 50. | 1,2,3,4-tetrahydro-isoquinolin-2-yl | CH₂ | H | 2 | 2 | H | H |
| 51. | pyrrolo[2,3-b]pyrid-1-yl | CH₂ | H | 2 | 2 | H | H |
| 52. | benzimidazol-1-yl | CH₂ | H | 2 | 2 | H | H |
| 53. | 4-phenylimidazol-1-yl | CH₂ | H | 2 | 2 | H | H |
| 54. | phenyl | (CH₂)₂ | H | 1 | 2 | H | CH₃ |
| 55. | 4-F-phenyl | (CH₂)₂ | H | 2 | 2 | H | CH₃ |
| 56. | 3-Cl-phenyl | (CH₂)₂ | H | 2 | 2 | H | CH₃ |
| 57. | 4-Cl-phenyl | (CH₂) ₂ | H | 2 | 2 | H | CH₃ |
| 58. | 3-CF₃-phenyl | (CH2) ₂ | H | 2 | 2 | H | CH₃ |
| 59. | 4-CF₃-phenyl, | (CH₂)₂ | H | 2 | 2 | H | CH₃ |
| 60. | 3-CN-phenyl | (CH)₂ | H | 2 | 2 | H | CH₃ |
| 61. | 4-CH₃-phenyl | (CH₂)₂ | H | 2 | 2 | H | H |
| 62. | 4-CH₃-phenyl | (CH₂)₂ | H | 2 | 2 | H | CH₃ |
| 63. | 4-CH₃O-phenyl | (CH₂)₂ | H | 2 | 2 | H | H |
| 64. | 4-CH₃O-phenyl | (CH₂)₂ | H | 2 | 2 | H | CH₃ |
| 65. | 2-phenylphenyl | (CH₂)₂ | H | 2 | 2 | H | CH₃ |
| 66. | 3-pherlylphenyl | (CH₂)₂ | H | 2 | 2 | H | CH₃ |
| 67. | naphth-1-yl | (CH2)2 | H | 2 | 2 | H | CH₃ |
| 68. | naphth-2-yl | (CH₂)₂ | H | 2 | 2 | H | CH₃ |
| 69. | pyrimidin-2-yl | (CH₂)₂ | H | 2 | 2 | H | CH₃ |
| 70. | pyrimidin-5-yl | (CH₂)₂ | H | 2 | 2 | H | CH₃ |
| 71. | 6-cyclopentylpyrid-2-yl | (CH₂)₂ | H | 2 | 2 | H | CH₃ |
| 72. | 6-(pyrrolidin-1-yl)-pyrid-2-yl | (CH₂)₂ | H | 2 | 2 | H | CH₃ |
| 73. | thiazol-2-yl | (CH₂)₂ | H | 2 | 2 | H | CH₃ |
| 74. | isoquinolin-1-yl | (CH₂)₂ | H | 2 | 2 | H | CH₃ |
| 75. | 1,2,3,4-tetrahydro-quinolin-1-yl | (CH₂)₂ | H | 2 | 2 | H | H |
| 76. | 1,2,3,4-tetrahydro-isoquinolin-2-yl | (CH₂)₂ | H | 2 | 2 | H | H |
| 77. | indol-1-yl | (CH₂)₂ | H | 2 | 2 | H | H |
| 78. | indolin-1-yl | (CH₂)₂ | H | 2 | 2 | H | H |
| 79. | pyrrolo[2,3-b]pyrid-1-yl | (CH₂)₂ | H | 2 | 2 | H | H |
| 80. | benzimidazol-1-yl | (CH₂)₂ | H | 2 | 2 | H | H |
| 81. | 4-phenylimidazol-1-yl | (CH₂)₂ | H | 2 | 2 | H | H |
| 82. | 3-Cl-phenyl | (CH₂)₃ | H | 2 | 2 | H | CH₃ |
| 83. | 4-Cl-phenyl | (CH₂)₃ | H | 2 | 2 | H | CH₃ |
| 84. | 3-CF₃-phenyl | (CH₂)₃ | H | 2 | 2 | H | CH₃ |
| 85. | 4-CF₃-phenyl | (CH₂)₃ | H | 2 | 2 | H | CH₃ |
| 86. | 3-CN-phenyl | (CH₂)₃ | H | 2 | 2 | H | CH₃ |
| 87. | 2-phenylphehyl | (CH₂)₃ | H | 2 | 2 | H | CH₃ |
| 88. | 3-phenylphenyl | (CH₂)₃ | H | 2 | 2 | H | CH₃ |
| 89. | naphth-1-yl | (CH₂)₃ | H | 2 | 2 | H | CH₃ |
| 90. | naphth-2-yl | (CH₂)₃ | H | 2 | 2 | H | CH₃ |
| 91. | pyrimidin-2-yl | (CH₂)₃ | H | 2 | 2 | H | CH₃ |
| 92. | pyrimidin-5-yl | (CH₂)₃ | H | 2 | 2 | H | CH₃ |
| 93. | thiazol-2-yl | (CH₂)₃ | H | 2 | 2 | H | CH₃ |
| 94. | 3-Cl-phenyl | C≡C | H | 2 | 2 | H | CH₃ |
| 95. | 4-Cl-phenyl | C≡C | H | 2 | 2 | H | CH₃ |
| 96. | 3-CF₃-phenyl | C≡C | H | 2 | 2 | H | CH₃ |
| 97. | 4-CF₃-phenyl | C=C | H | 2 | 2 | H | CH₃ |
| 98. | 3-CN-phenyl | C≡C | H | 2 | 2 | H | CH₃ |
| 99. | 2-phenylphenyl | C≡C | H | 2 | 2 | H | CH₃ |
| 100. | 3-phenylphenyl | C≡C | H | 2 | 2 | H | CH₃ |
| 101. | naphth-1-yl | C≡C | H | 2 | 2 | H | CH₃ |
| 102. | naphth-2-yl | C≡C | H | 2 | 2 | H | CH₃ |
| 103. | pyrimidin-2-yl | C≡C | H | 2 | 2 | H | CH₃ |
| 104. | pyrimidin-5-yl | C≡C | H | 2 | 2 | H | CH₃ |
| 105. | thiazol-2-yl | C≡C | H | 2 | 2 | H | CH₃ |
| 106. | 3-Cl-phenyl | C≡CCH₂ | H | 2 | 2 | H | CH₃ |
| 107. | 4-Cl-phenyl | C≡CCH₂ | H | 2 | 2 | H | CH₃ |
| 108. | 3-CF3-phenyl | C≡CCH₂ | H | 2 | 2 | H | CH₃ |
| 109. | 4-CF₃-phenyl | C≡CCH₂ | H | 2 | 2 | H | CH₃ |
| 110. | 3-CN-phenyl | C≡CCH₂ | H | 2 | 2 | H | CH₃ |
| 111. | 2-phenylphenyl | C≡CCH₂ | H | 2 | 2 | H | CH₃ |
| 112. | 3-phenylphenyl | C≡CCH₂, | H | 2 | 2 | H | CH₃ |
| 113. | naphth-1₋yl | C≡CCH₂ | H | 2 | 2 | H | CH₃ |
| 114. | naphth-2-yl | C≡CCH₂ | H | 2 | 2 | H | CH₃ |
| 115. | pyrimidin-2-yl | C≡CCH₂ | H | 2 | 2 | H | CH₃ |
| 116. | pyrimidin-5-yl | C≡CCH₂ | H | 2 | 2 | H | CH₃ |
| 117. | thiazol-2-yl | C≡CCH₂ | H | 2 | 2 | H | CH₃ |
| 118. | 6-(pyrrolidin-1-yl)-pyrid-2-yl | CH₂ | H | 2 | 2 | H | CH₃ |
| 119 . | 6-(1-isoptopylpiperidin-4-yl)pyrid-2-yl | (CH₂)₂ | H | 2 | 2 | H | CH₃ |
in the base form or in the form of an addition salt with an acid, of a hydrate or of a solvate.

2. Process for the preparation of a compound of formula (I) according to Claim 1, comprising the stage consisting in converting the carbamate-ester of general formula (IV) 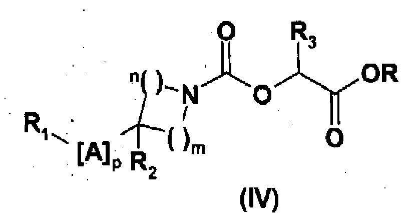 in which R₁, A, R₂, R₃, p, m and n are as defined in the formula (I) according to Claim 1 and R represents a methyl or ethyl group,
by aminolysis using an amine of general formula R₄NH₂, in which R₄ is as defined in the formula (I) according to Claim 1.

3. Compound corresponding to the general formula (IV) 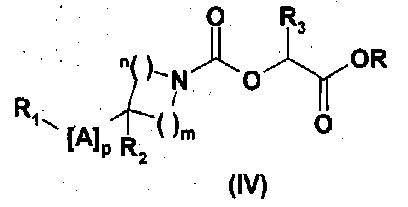 in which R₁, A, R₂, R₃, p, m and n are as defined in the formula (I) according to Claim 1 and R represents a methyl or ethyl group.

4. Medicament comprising a compound of formula (I) according to Claim 1, or a pharmaceutically acceptable salt or hydrate or solvate of the compound of formula (I).

5. Pharmaceutical composition comprising at least one compound of formula (I) according to Claim 1, in the base or pharmaceutically acceptable salt, hydrate or solvate form, and optionally one or more pharmaceutically acceptable excipients.

6. Compound of formula (I) according to Claim 1, in the base or pharmaceutically acceptable salt, hydrate or solvate form, for its use as medicament.

7. Use of a compound of formula (I) according to Claim 1, in the base or pharmaceutically acceptable salt, hydrate or solvate form, in the preparation of a medicament intended to prevent or treat acute or chronic pain, dizziness, vomiting, nausea, eating disorders, neurological and psychiatric pathologies, acute or chronic neurodegenerative diseases, epilepsy, sleep disorders, cardiovascular diseases or renal ischaemia.

## Patentansprüche

1. Verbindung der Formel (I): 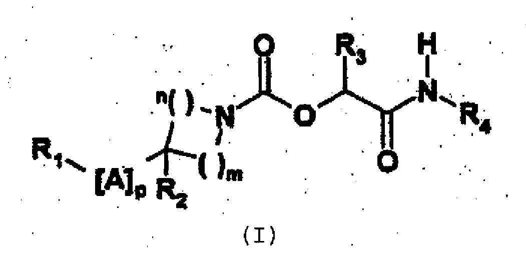 ausgewählt unter den Verbindungen der folgenden Tabelle:
| **Nr.** | **R₁** | **[A] ₚ** | **R₂** | **n** | **m** | **R₃** | **R₄** |
|---|---|---|---|---|---|---|---|
| 1. | Phenyl | Bindung | H | 2 | 2 | H | H |
| 2. | Phenyl | Bindung | H | 2 | 2 | H | CH₃ |
| 3. | 3-CF₃-Phenyl | Bindung | H | 2 | 2 | H | H |
| 4. | 3-CF₃-Phenyl | Bindung | H | 2 | 2 | H | CH₃ |
| 5. | 5-Isobutylpyridin-2-yl | Bindung | H | 2 | 2 | H | CH₃ |
| 6. | 6-Isobutylpyridin-2-yl | Bindung | H | 2 | 2 | H | CH₃ |
| 7. | 6-Cyclopentylpyridin-2-yl | Bindung | H | 2 | 2 | H | CH₃ |
| 8. | 5-(4-F-Phenyl)pyridin-2-yl | Bindung | H | 2 | 2 | H | CH₃ |
| 9. | 6-(4-F-Phenyl)pyridin-2-yl | Bindung | H | 2 | 2 | H | CH₃ |
| 10. | 6-(4-Cl-Phenyl)pyridin-2-yl | Bindung | H | 2 | 2 | H | CH₃ |
| 11. | 5- (4-CF₃-Phenyl)pyridin-2-yl | Bindung | H | 2 | 2 | H | CH₃ |
| 12. | 6-(4-CF₃-Phenyl)pyrzdin-2-yl | Bindung | H | 2 | 2 | H | CH₃ |
| 13. | 5- (3-CF₃-Phenyl)-1-methyl-pyrazol-3-yl | Bindung | H | 2 | 2 | H | CH₃ |
| 14. | 4-Phenylimidazol-1-yl | Bindung | H | 2 | 2 | H | CH₃ |
| 15. | 5-Phenyl-1,3,4-oxadiazol-2-yl | Bindung | H | 2 | 2 | H | H |
| 16. | 5 -Phenyl-1,3,4-oxadiazol-2-yl | Bindung | H | 2 | 2 | H | CH₃ |
| 17. | 5-(4-F-Phenyl)-1,3,4-oxa-diazol-2-yl | Bindung | H | 2 | 2 | H | H |
| 18. | 5-(4-F-Phenyl)-1,3,4-oxa-diazol-2-yl | Bindung | H | 2 | 2 | H | CH₃ |
| 19. | 5-(3-CF₃-Phenyl)-1,3,4-oxadiazol-2-yl | Bindung | H | 2 | 2 | H | H |
| 20. | 5- (3-CF₃-Phenyl)-1,3,4-oxadiazol-2-yl | Bindung | H | 2 | 2 | H | CH₃ |
| 21. | 3-(3-CF₃-Phenyl)-1,2,4-oxadiazol-5-yl | Bindung | H | 2 | 2 | H | H |
| 22. | 3-(3-CF₃-Phenyl)-1,2,4-oxadiazol-5-yl | Bindung | H | 2 | 2 | H | CH₃ |
| 23. | Benzoxazol-2-yl | Bindung | H | 2 | 2 | H | CH₃ |
| 24. | Benzothiazol-2-yl | Bindung | H | 2 | 2 | H | H |
| 25. | Benzothiazol-2-yl | Bindung | H | 2 | 2 | H | CH₃ |
| 26. | Benzimidazol-2-yl | Bindung | H | 2 | 2 | H | CH₃ |
| 27. | Benzimidazol-1-yl | Bindung | H | 2 | 2 | H | H |
| 28. | 2-Phenylbenzimidazol-1-yl | Bindung | H | 2 | 2 | H | CH₃ |
| 29. | Benzotriazol-1-yl | Bindung | H | 2 | 2 | H | CH₃ |
| 30. | 5-CF₃-Benzotriazol-1-yl | Bindung | H | 2 | 2 | H | H |
| 31. | Indol-1-yl | Bindung | H | 2 | 2 | H | H |
| 32. | 4-Br-Phenyl | Bindung | OH | 2 | 2 | H | CH₃ |
| 33. | 4-(4-F-Phenyl)phenyl | Bindung | OH | 2 | 2 | H | CH₃ |
| 34. | 4-(4-Cl-Phenyl)phenyl | Bindung | OH | 2 | 2 | H | CH₃ |
| 35. | 4-(4-CH₃-Phenyl)phenyl | Bindung | OH | 2 | 2 | H | CH₃ |
| 36. | 4-(4-(n-Butyl)phenyl)-phenyl | Bindung | OH | 2 | 2 | H | CH₃ |
| 37. | 4-(4-CF₃-Phenyl)phenyl | Bindung | OH | 2 | 2 | H | CH₃ |
| 38. | 4 - (4-CH₃O-Phenyl) phenyl | Bindung | OH | 2 | 2 | H | CH₃ |
| 39. | 4- (4-C₂H₅O-Phenyl) phenyl | Bindung | OH | 2 | 2 | H | CH₃ |
| 40. | 4-(3-Cl, 4-Cl-Phenyl)phenyl | Bindung | OH | 2 | 2 | H | CH₃ |
| 41. | 4- (3-F, 4-CH₃O-Phenyl) - phenyl | Bindung | OH | 2 | 2 | H | CH₃ |
| 42. | 4-(3-Cl, 4-F-Phenyl)phenyl | Bindung | OH | 2 | 2 | H | CH₃ |
| 43. | Naphthalin-2-yl | CH₂ | H | 2 | 2 | H | CH₃ |
| 44. | 4-Phenylphenyl | CH₂ | H | 2 | 2 | H | CH₃ |
| 45. | 6-Cyclopentylpyridin-2-yl | CH₂ | H | 2 | 2 | H | CH₃ |
| 46. | 6- (4-F-Phenyl)pyridin-2-yl | CH₂ | H | 2 | 2 | H | CH₃ |
| 47. | Indol-1-yl | CH₂ | H | 2 | 2 | H | H |
| 48. | Indolin-1-yl | CH₂ | H | 2 | 2 | H | H |
| 49. | 1,2,3,4-Tetrahydro-chinolin-1-yl | CH₂ | H | 2 | 2 | H | H |
| 50. | 1,2,3,4-Tetrahydroiso-chinolin-2-yl | CH₂ | H | 2 | 2 | H | H |
| 51. | Pyrrolo[2,3-b]pyrid-1-yl | CH₂ | H | 2 | 2 | H | H |
| 52. | Benzimidazol-1-yl | CH₂ | H | 2 | 2 | H | H |
| 53. | 4-Phenylimidazol-1-yl | CH₂ | H | 2 | 2 | H | H |
| 54. | Phenyl | (CH₂)₂ | H | 1 | 2 | H | CH₃ |
| 55. | 4-F-Phenyl | (CH₂)₂ | H | 2 | 2 | H | CH₃ |
| 56. | 3-Cl-Phenyl | CH₂)₂ | H | 2 | 2 | H | CH₃ |
| 57. | 4-Cl-Phenyl | (CH₂)₂ | H | 2 | 2 | H | CH₃ |
| 58. | 3-CF₃-Phenyl | (CH₂)₂ | H | 2 | 2 | H | CH₃ |
| 59. | 4-CF₃-Phenyl | (CH₂)₂ | H | 2 | 2 | H | CH₃ |
| 60. | 3-CN-Phenyl | (CH₂)₂ | H | 2 | 2 | H | CH₃ |
| 61. | 4-CH₃-Phenyl | (CH₂) ₂ | H | 2 | 2 | H | H |
| 62. | 4-CH₃-Phenyl | CH₂)₂ | H | 2 | 2 | H | CH₃ |
| 63. | 4-CH₃O-Phenyl | (CH₂)₂ | H | 2 | 2 | H | H |
| 64. | 4-CH₃O-Phenyl | (CH₂) ₂ | H | 2 | 2 | H | CH₃ |
| 65. | 2-Phenylphenyl | (CH₂)₂ | H | 2 | 2 | H | CH₃ |
| 66. | 3-Phenylphenyl | (CH₂) ₂ | H | 2 | 2 | H | CH₃ |
| 67. | Naphthalin-1-yl | (CH₂) ₂ | H | 2 | 2 | H | CH₃ |
| 68. | Naphthalin-2-yl | (CH₂)₂ | H | 2 | 2 | H | CH₃ |
| 69. | Pyrimidin-2-yl | (CH₂)₂ | H | 2 | 2 | H | CH₃ |
| 70. | Pyrimidin-5-yl | (CH₂) ₂ | H | 2 | 2 | H | CH₃ |
| 71. | 6-Cyclopentylpyridin-2-yl | (CH₂) ₂ | H | 2 | 2 | H | CH₃ |
| 72. | 6-(Pyrrolidin-1-yl)-pyridin-2-yl | (CH₂) ₂ | H | 2 | 2 | H | CH₃ |
| 73. | Thiazol-2-yl | (CH₂) ₂ | H | 2 | 2 | H | CH₃ |
| 74. | Isochinolin-1-yl | CH₂) ₂ | H | 2 | 2 | H | CH₃ |
| 75. | 1,2,3,4-Tetrahydro-chinolin-1-yl | (CH₂)₂ | H | 2 | 2 | H | H |
| 76. | 1,2,3,4-tetrahydroiso-chinolin-2-yl | (CH₂) ₂ | H | 2 | 2 | H | H |
| 77. | Indol-1-yl | (CH₂) ₂ | H | 2 | 2 | H | H |
| 78. | Indolin-1-yl | (CH₂) ₂ | H | 2 | 2 | H | H |
| 79. | Pyrrolo[2,3-b]pyridin-1-yl | (CH₂)₂ | H | 2 | 2 | H | H |
| 80. | Benzimidazol-1-yl | (CH₂) ₂ | H | 2 | 2 | H | H |
| 81. | 4-Phenylimidazol-1-yl | (CH₂) ₂ | H | 2 | 2 | H | H |
| 82. | 3-Cl-Phenyl | (CH₂) ₃ | H | 2 | 2 | H | CH₃ |
| 83. | 4-Cl-Phenyl | (CH₂) ₃ | H | 2 | 2 | H | CH₃ |
| 84. | 3-CF₃-Phenyl | (CH₂) ₃ | H | 2 | 2 | H | CH₃ |
| 85. | 4-CF₃-Phenyl | (CH₂) ₃ | H | 2 | 2 | H | CH₃ |
| 86. | 3-CN-Phenyl | (CH₂) ₃ | H | 2 | 2 | H | CH₃ |
| 87. | 2-Phenylphenyl | (CH₂) ₃ | H | 2 | 2 | H | CH₃ |
| 88. | 3-Phenylphenol | (CH₂) ₃ | H | 2 | 2 | H | CH₃ |
| 89. | Naphthalin-1-yl | (CH₂) ₃ | H | 2 | 2 | H | CH₃ |
| 90. | Naphthalin-2-yl | (CH₂) ₃ | H | 2 | 2 | H | CH₃ |
| 91. | Pyrimidin-2-yl | (CH₂) ₃ | H | 2 | 2 | H | CH₃ |
| 92. | Pyrimidin- 5₋yl | (CH₂) ₃ | H | 2 | 2 | H | CH₃ |
| 93. | Thiazol-2-yl | (CH₂) ₃ | H | 2 | 2 | H | CH₃ |
| 94. | 3-Cl-Phenyl | C≡C | H | 2 | 2 | H | CH₃ |
| 95. | 4-Cl-Phenyl | C=C | H | 2 | 2 | H | CH₃ |
| 95. | 3-CF₃-Phenyl | C=C | H | 2 | 2 | H | CH₃ |
| 97. | 4-CF₃-Phenyl | C≡C | H | 2 | 2 | H | CH₃ |
| 98. | 3-CN-Phenyl | C=C | H | 2 | 2 | H | CH₃ |
| 99. | 2-Phenylphenyl | C≡C | H | 2 | 2 | H | CH₃ |
| 100. | 3-Phenylphenyl | C≡C | H | 2 | 2 | H | CH₃ |
| 101. | Naphthalin-1-yl | C≡C | H | 2 | 2 | H | CH₃ |
| 102. | Naphthalin-2-yl | C≡C | H | 2 | 2 | H | CH₃ |
| 103. | Pyrimidin-2-yl | C≡C | H | 2 | 2 | H | CH₃ |
| 104. | Pyrimidin-5-yl | C≡C | H | 2 | 2 | H | CH₃ |
| 105. | Thiazol-2-yl | C≡C | H | 2 | 2 | H | CH₃ |
| 106. | 3-Cl-Phenyl | C≡CCH₂ | H | 2 | 2 | H | CH₃ |
| 107. | 4-Cl-Phenyl | C≡CCH₂ | H | 2 | 2 | H | CH₃ |
| 108. | 3-CF₃-Phenyl | C≡CCH₂ | H | 2 | 2 | H | CH₃ |
| 109. | 4-CF₃-Phenyl | C≡CCH₂ | H | 2 | 2 | H | CH₃ |
| 110. | 3-CN-Phenyl | C≡CCH₂ | H | 2 | 2 | H | CH₃ |
| 111. | 2-Phenylphenyl | C≡CCH₂ | H | 2 | 2 | H | CH₃ |
| 112. | 3-Phenylphenyl | C≡CCH₂ | H | 2 | 2 | H | CH₃ |
| 113. | Naphthalin-1-yl | C≡CCH₂ | H | 2 | 2 | H | CH₃ |
| 114. | Naphthalin-2-yl | C≡CCH₂ | H | 2 | 2 | H | CH₃ |
| 115. | Pyrimidin-2-yl | C≡CCH₂ | H | 2 | 2 | H | CH₃ |
| 116. | Pyrimidin-5-yl | C≡CCH₂ | H | 2 | 2 | H | CH₃ |
| 117. | Thiazol-2-yl | C≡CCH₂ | H | 2 | 2 | H | CH₃ |
| 118. | 6-(Pyrrolidin-1-yl)-pyridin-2-yl | CH₂ | H | 2 | 2 | H | CH₃ |
| 119. | 6-(1-Isopropylpiperidin-4-yl)pyridin-2-yl | (CH₂)₂ | H | 2 | 2 | H | CH₃ |
in Basen-, Säureadditionssalz-, Hydrat- oder Solvatform.

2. Verfahren zur Herstellung einer Verbindung der Formel (I) nach Anspruch 1, bei dem man den Carbamatester der allgemeinen Formel (IV) 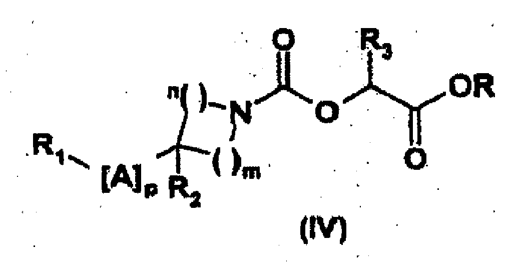 worin R₁, A, R₂, R₃, p, m und n die in der allgemeinen Formel (I) nach Anspruch 1 angegebene Bedeutung besitzen und R für eine Methyl- oder Ethylgruppe steht,
durch Aminolyse mit einem Amin der allgemeinen Formel R₄NH₂, worin R₄ die in der allgemeinen Formel (I) nach Anspruch 1 angegebene Bedeutung besitzt, umwandelt.

3. Verbindung der allgemeinen Formel (IV) 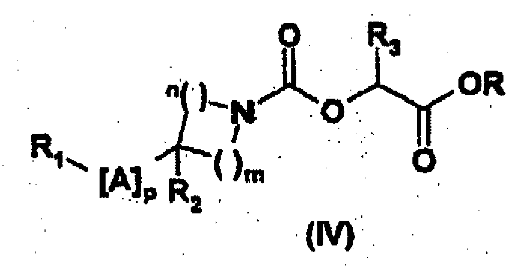 worin R₁, A, R₂, R₃, p, m und n die in der allgemeinen Formel (I) nach Anspruch 1 angegebene Bedeutung besitzen und R für eine Methyl- oder Ethylgruppe steht.

4. Arzneimittel, umfassend mindestens eine Verbindung der Formel (I) nach Anspruch 1 oder ein pharmazeutisch unbedenkliches Salz oder auch ein pharmazeutisch unbedenkliches Hydrat oder Solvat der Verbindung der Formel (I).

5. Pharmazeutische Zusammensetzung, enthaltend mindestens eine Verbindung der Formel (I) nach Anspruch 1 in pharmazeutisch unbedenklicher Basen-, Salz-, Hydrat- oder Solvatform und gegebenenfalls einen oder mehrere pharmazeutisch unbedenkliche Hilfsstoffe.

6. Verbindung der Formel (I) nach Anspruch 1 in pharmazeutisch unbedenklicher Basen-, Salz-, Hydrat- oder Solvatform zur Verwendung als Arzneimittel.

7. Verwendung einer Verbindung der Formel (I) nach Anspruch 1 in pharmazeutisch unbedenklicher Basen-, Salz-, Hydrat- oder Solvatform zur Herstellung eines Arzneimittels zur Prävention oder Behandlung von akuten oder chronischen Schmerzen, Schwindel, Erbrechen, Übelkeit, Eßstörungen, neurologischen und psychiatrischen Pathologien, akuten oder chronischen neurodegenerativen Erkrankungen, Epilepsie, Schlafstörungen, Herz-Kreislauf-Erkrankungen, Nierenischämie.
